(19) Europäisches Patentamt
European Patent Office
Office européen des brevets

(11) EP 2 091 950 B1

(12) EUROPEAN PATENT SPECIFICATION

(45) Date of publication and mention of the grant of the patent:
**22.09.2010 Bulletin 2010/38**

(21) Application number: **07857804.4**

(22) Date of filing: **19.12.2007**

(51) Int Cl.:
***C07D 413/10*** *(2006.01)* ***C07D 413/14*** *(2006.01)*
***A61K 31/4245*** *(2006.01)* ***A61P 37/00*** *(2006.01)*

(86) International application number:
**PCT/EP2007/064181**

(87) International publication number:
**WO 2008/074820 (26.06.2008 Gazette 2008/26)**

(54) **OXADIAZOLE DERIVATIVES AS S1P1 RECEPTOR AGONISTS**

OXADIAZOLDERIVATE ALS S1P1-REZEPTOR-AGONISTEN

DÉRIVÉS D'OXADIAZOLE EN TANT QU'AGONISTES DU RÉCEPTEUR S1P1

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**HR**

(30) Priority: **21.12.2006 GB 0625648**

(43) Date of publication of application:
**26.08.2009 Bulletin 2009/35**

(73) Proprietor: **Glaxo Group Limited**
**Greenford Middlesex UB6 0NN (GB)**

(72) Inventors:
- **AHMED, Mahmood**
  **Singapore 138667 (SG)**
- **MYATT, James**
  **Harlow Essex CM19 5AW (GB)**
- **NORTON, David**
  **Harlow Essex CM19 5AW (GB)**
- **RIVERS, Dean Andrew**
  **Singapore 138667 (SG)**

(74) Representative: **Griffith, Johanna Elise**
**GlaxoSmithKline**
**Corporate Intellectual Property**
**(CN9.25.1)**
**980 Great West Road**
**Brentford, Middlesex TW8 9GS (GB)**

(56) References cited:
**WO-A-03/105771**
**WO-A-2004/103279**
**WO-A-2005/032465**
**WO-A-2006/131336**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 091 950 B1

## Description

The present invention relates to novel oxadiazole derivatives having pharmacological activity, processes for their preparation, pharmaceutical compositions containing them and their use in the treatment of various disorders.

Sphingosine 1-phosphate (S1P) is a bioactive lipid mediator formed by the phosphorylation of sphingosine by sphingosine kinases and is found in high levels in the blood. It is produced and secreted by a number of cell types, including those of hematopoietic origin such as platelets and mast cells (Okamoto et al 1998 J Biol Chem 273(42):27104; Sanchez and Hla 2004, J Cell Biochem 92:913). It has a wide range of biological actions, including regulation of cell proliferation, differentiation, motility, vascularisation, and activation of inflammatory cells and platelets (Pyne and Pyne 2000, Biochem J. 349: 385). Five subtypes of S1 P responsive receptor have been described, S1P1 (Edg-1), S1P2 (Edg-5), S1P3 (Edg-3), S1P4 (Edg-6), and S1 P5 (Edg-8), forming part of the G-protein coupled endothelial differentiation gene family of receptors (Chun et al 2002 Pharmacological Reviews 54:265, Sanchez and Hla 2004 J Cellular Biochemistry, 92:913). These 5 receptors show differential mRNA expression, with S1P1-3 being widely expressed, S1 P4 expressed on lymphoid and hematopoietic tissues and S1 P5 primarily in brain and to a lower degree in spleen. They signal via different subsets of G proteins to promote a variety of biological responses (Kluk and Hla 2002 Biochem et Biophysica Acta 1582:72, Sanchez and Hla 2004, J Cellular Biochem 92:913).

Proposed roles for the S1P1 receptor include lymphocyte trafficking, cytokine induction/suppression and effects on endothelial cells (Rosen and Goetzl 2005 Nat Rev Immunol. 5:560). Agonists of the S1 P1 receptor have been used in a number of autoimmune and transplantation animal models, including Experimental Autoimmune Encephalomelitis (EAE) models of MS, to reduce the severity of the induced disease (Brinkman et al 2003 JBC 277:21453; Fujino et al 2003 J Pharmacol Exp Ther 305:70; Webb et al 2004 J Neuroimmunol 153:108; Rausch et al 2004 J Magn Reson Imaging 20:16). This activity is reported to be mediated by the effect of S1 P1 agonists on lymphocyte circulation through the lymph system. Treatment with S1 P1 agonists results in the sequestration of lymphocytes within secondary lymphoid organs such as the lymph nodes, inducing a reversible peripheral lymphopoenia in animal models (Chiba et al 1998, J Immunology 160:5037, Forrest et al 2004 J Pharmacol Exp Ther 309:758; Sanna et al 2004 JBC 279:13839). Published data on agonists suggests that compound treatment induces loss of the S1P1 receptor from the cell surface via internalisation (Graler and Goetzl 2004 FASEB J 18:551; Matloubian et al 2004 Nature 427:355; Jo et al 2005 Chem Biol 12:703) and it is this reduction of S1P1 receptor on immune cells which contributes to the reduction of movement of T cells from the lymph nodes back into the blood stream.

S1P1 gene deletion causes embryonic lethality. Experiments to examine the role of the S1P1 receptor in lymphocyte migration and trafficking have included the adoptive transfer of labelled S1 P1 deficient T cells into irradiated wild type mice. These cells showed a reduced egress from secondary lymphoid organs (Matloubian et al 2004 Nature 427:355).

S1P1 has also been ascribed a role in endothelial cell junction modulation (Allende et al 2003 102:3665, Blood Singelton et al 2005 FASEB J 19:1646). With respect to this endothelial action, S1P1 agonists have been reported to have an effect on isolated lymph nodes which may be contributing to a role in modulating immune disorders. S1P1 agonists caused a closing of the endothelial stromal 'gates' of lymphatic sinuses which drain the lymph nodes and prevent lymphocyte egress (Wei wt al 2005, Nat. Immunology 6:1228).

The immunosuppressive compound FTY720 (JP11080026-A ) has been shown to reduce circulating lymphocytes in animals and man, have disease modulating activity in animal models of immune disorders and reduce remission rates in relapsing remitting Multiple Sclerosis (Brinkman et al 2002 JBC 277:21453, Mandala et al 2002 Science 296:346, Fujino et al 2003 J Pharmacology and Experimental Therapeutics 305:45658, Brinkman et al 2004 American J Transplantation 4:1019, Webb et al 2004 J Neuroimmunology 153:108, Morris et al 2005 EurJ Immunol 35: 3570, Chiba 2005 Pharmacology and Therapeutics 108:308, Kahan et al 2003, Transplantation 76:1079, Kappos et al 2006 New Eng J Medicine 335:1124). This compound is a prodrug that is phosphorylated in vivo by sphingosine kinases to give a molecule that has agonist activity at the S1P1, S1P3, S1 P4 and S1 P5 receptors. Clinical studies have demonstrated that treatment with FTY720 results in bradycardia in the first 24 hours of treatment (Kappos et al 2006 New Eng J Medicine 335:1124). The bradycardia is thought to be due to agonism at the S1 P3 receptor, based on a number of cell based and animal experiments. These include the use of S1P3 knockout animals which, unlike wild type mice, do not demonstrate bradycardia following FTY720 administration and the use of S1P1 selective compounds. (Hale et al 2004 Bioorganic & Medicinal Chemistry Letters 14:3501, Sanna et al 2004 JBC 279:13839, Koyrakh et al 2005 American J Transplantation 5:529).

Hence, there is a need for S1 P1 receptor agonist compounds with selectivity over S1 P3 which might be expected to show a reduced tendency to induce bradycardia.

The following patent applications describe oxadiazole derivatives as S1 P1 agonists: WO03/105771, WO05/058848, WO06/047195, WO06/100633, WO06/115188 WO06/131336, W02007/024922 and WO07/116866. WO 05/032465 and WO 04/103279 also disclose oxadiazole derivatives as S1P1 agonists. A structurally novel class of compounds has now been found which provides agonists of the S1P1 receptor.

The present invention therefore provides compounds of formula (I) or pharmaceutically acceptable salts or solvates thereof:

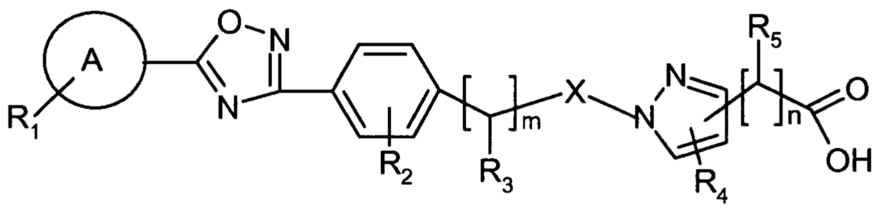

(I)

wherein:

A is a phenyl or a 5 or 6-membered heteroaryl ring;
$R_1$ is hydrogen or up to three substituents selected from halogen, $C_{(1-4)}$alkyl, $C_{(5-6)}$cycloalkyl, $C_{(3-6)}$cycloalkenyl, $C_{(1-4)}$alkoxy, trifluoromethoxy, difluoromethoxy, trifluoromethyl, cyano, optionally substituted phenyl or optionally substituted 5 or 6 membered heteroaryl rings;
$R_2$ is hydrogen or up to three substituents selected from halogen, $C_{(1-4)}$alkyl, $C_{(1-4)}$alkoxy, trifluoromethoxy, difluoromethoxy, trifluoromethyl or cyano;
$R_3$, $R_4$ and $R_5$ are each independently selected from hydrogen, fluoro and methyl;
X is an optionally substituted $CH_2$ or a direct bond;
m is 0 to 1; and
n is 0 to 2.

When $R_1$ is phenyl or a 5 or 6 membered heteroaryl ring it may be substituted by up to three substituents selected from halogen, $C_{(1-4)}$alkyl, $C_{(1-4)}$alkoxy, trifluoromethoxy, difluoromethoxy, trifluoromethyl and cyano.

When X is $CH_2$ it may be substituted by fluoro or methyl.

The term "alkyl" as a group or part of a group e.g. alkoxy or hydroxyalkyl refers to a straight or branched alkyl group in all isomeric forms. The term "$C_{(1-4)}$ alkyl" refers to an alkyl group, as defined above, containing at least 1, and at most 4 carbon atoms Examples of such alkyl groups include methyl, ethyl, propyl, *iso*-propyl, *n*-butyl, *iso*butyl, *sec*-butyl, or *tert*-butyl, Examples of such alkoxy groups include methoxy, ethoxy, propoxy, *iso*-propoxy, butoxy, *iso*-butoxy, *sec*-butoxy and *tert*-butoxy.

Suitable $C_{(3-6)}$cycloalkyl groups include cyclopropyl, cyclobutyl, cyclopentyl and cyclohexyl.

Suitable $C_{(5-6)}$cycloalkenyl groups include cyclopentenyl and cyclohexenyl.

As used herein, the term "halogen" refers to fluorine (F), chlorine (Cl), bromine (Br), or iodine (I) and the term "halo" refers to the halogen: fluoro (-F), chloro (-Cl), bromo(-Br) and iodo(-I).

The term "heteroaryl" represents an unsaturated ring which comprises one or more heteroatoms. When the term heteroaryl represents a 5 membered group it contains a heteroatom selected from O, N or S and may optionally contain a further 1 to 3 nitrogen atoms. When heteroaryl represents a 6-membered group it contains from 1 to 3 nitrogen atoms. Examples of such 5 or 6 membered heteroaryl rings include pyrrolyl, triazolyl, thiadiazolyl, tetrazolyl, imidazolyl, pyrazolyl, isothiazolyl, thiazolyl, isoxazolyl, oxazolyl, oxadiazolyl, furazanyl, furanyl, thienyl, pyridyl, pyrimidinyl, pyrazinyl, pyridazinyl and triazinyl.

In one embodiment of the invention,

A is optionally substituted pyridyl, phenyl or thiophene; and/or
$R_1$ is optionally substituted phenyl, trifluoromethyl, trifluoromethoxy, chloro, isopropoxy, isobutyl, methoxy, cyano, cyclohexyl, cyclohexenyl or ethyl.; and/or
$R_2$ is hydrogen, chloro or bromo; and/or
$R_3$, $R_4$ and $R_5$ are each hydrogen; and/or
X is $CH_2$; and/or
m is 0; and/or
n is 0.

In one embodiment of the invention,

A is optionally substituted thiophene; and/or
$R_1$ is hydrogen or trifluoromethyl; and/or
$R_2$, $R_3$, $R_4$ and $R_5$ are each hydrogen; and/or
X is $CH_2$; and/or
m is 0; and/or
n is 0.

In another embodiment of the invention,

A is thiophene substituted by phenyl; and/or
$R_1$ is hydrogen or trifluoromethyl; and/or
$R_2$, $R_3$, $R_4$ and $R_5$ are each hydrogen; and/or
X is $CH_2$; and/or
m is 0; and/or
n is 0.

In certain of the compounds of formula (I), dependent upon the nature of the substituent there are chiral carbon atoms and therefore compounds of formula (I) may exist as stereoisomers. The invention extends to all optical isomers such as stereoisomeric forms of the compounds of formula (I) including enantiomers, diastereoisomers and mixtures thereof, such as racemates. The different stereoisomeric forms may be separated or resolved one from the other by conventional methods or any given isomer may be obtained by conventional stereoselective or asymmetric syntheses.
Certain of the compounds herein can exist in various tautomeric forms and it is to be understood that the invention encompasses all such tautomeric forms.
Suitable compounds of the invention are:

1-[(4-{5-[4-Phenyl-5-(trifluoromethyl)-2-thienyl]-1,2,4-oxadiazol-3-yl}phenyl)methyl]-1*H*-pyrazole-4-carboxylic acid

1-[(4-{5-[4-Phenyl-5-(trifluoromethyl)-2-thienyl]-1,2,4-oxadiazol-3-yl}phenyl)methyl]-1*H*-pyrazole-5-carboxylic acid

1-[(4-{5-[4-Phenyl-5-(trifluoromethyl)-2-thienyl]-1,2,4-oxadiazol-3-yl}phenyl)methyl]-1*H*-pyrazole-3-carboxylic acid

1-[(4-{5-[4-(4-fluorophenyl)-5-(trifluoromethyl)-2-thienyl]-1,2,4-oxadiazol-3-yl}phenyl)methyl]-1*H*-pyrazole-3-carboxylic acid

1-{[4-(5-{5-chloro-6-[(1-methylethyl)oxy]-3-pyridinyl}-1,2,4-oxadiazol-3-yl)phenyl]methyl}-1*H*-pyrazole-3-carboxylic acid

1-[(4-{5-[4-(methyloxy)-3-(trifluoromethyl)phenyl]-1,2,4-oxadiazol-3-yl}phenyl)methyl]-1*H*-pyrazole-3-carboxylic acid

1-{[4-(5-{3-chloro-4-[(1-methylethyl)oxy]phenyl}-1,2,4-oxadiazol-3-yl)phenyl]methyl}-1*H*-pyrazole-3-carboxylic acid

1-{[4-(5-{3-chloro-4-[(trifluoromethyl)oxy]phenyl}-1,2,4-oxadiazol-3-yl)phenyl]methyl}-1*H*-pyrazole-3-carboxylic acid

1-{[4-(5-{3-cyano-4-[(1-methylethyl)oxy]phenyl}-1,2,4-oxadiazol-3-yl)phenyl]methyl}-1*H*-pyrazole-3-carboxylic acid

1-[(4-{5-[2'-fluoro-2-(trifluoromethyl)-4-biphenylyl]-1,2,4-oxadiazol-3-yl}phenyl)methyl]-1H-pyrazole-3-carboxylic acid

1-[(4-{5-[2'-fluoro-2-(trifluoromethyl)-4-biphenylyl]-1,2,4-oxadiazol-3-yl}phenyl)methyl]-1H-pyrazole-3-carboxylic acid

1-[(4-{5-[4-cyclohexyl-3-(trifluoromethyl)phenyl]-1,2,4-oxadiazol-3-yl}phenyl)methyl]-1H-pyrazole-3-carboxylic acid

1-[(4-{5-[4-(1-cyclohexen-1-yl)-3-(trifluoromethyl)phenyl]-1,2,4-oxadiazol-3-yl}phenyl)methyl]-1H-pyrazole-3-carboxylic acid

1-[(4-{5-[4-(2-methylpropyl)-3-(trifluoromethyl)phenyl]-1,2,4-oxadiazol-3-yl}phenyl)methyl]-1H-pyrazole-3-carboxylic acid

1-({4-[5-(2-ethyl-4-biphenylyl)-1,2,4-oxadiazol-3-yl]phenyl}methyl)-1*H*-pyrazole-3-carboxylic acid

1-[(4-{5-[4-[(1-methylethyl)oxy]-3-(trifluoromethyl)phenyl]-1,2,4-oxa-diazol-3-yl}phenyl)methyl]-1*H*-pyrazole-3-carboxylic acid

1-({4-[5-(2-ethyl-2'-fluoro-4-biphenylyl)-1,2,4-oxadiazol-3-yl]phenyl} methyl)-1*H*-pyrazole-3-carboxylic acid

1-[(4-{5-[2-(trifluoromethyl)-4-biphenylyl]-1,2,4-oxadiazol-3-yl}phenyl) methyl]-1*H*-pyrazole-3-carboxylic acid

1-[(3-chloro-4-{5-[4-phenyl-5-(trifluoromethyl)-2-thienyl]-1,2,4-oxadiazol-3-yl}phenyl)methyl]-1H-pyrazole-3-carboxylic acid

1-[(2-chloro-4-{5-[4-phenyl-5-(trifluoromethyl)-2-thienyl]-1,2,4-oxadiazol-3-yl}phenyl)methyl]-1H-pyrazole-3-carboxylic acid

1-{[3-bromo-4-(5-{3-cyano-4-[(1-methylethyl)oxy]phenyl}-1,2,4-oxadiazol-3-yl)phenyl]methyl}-1H-pyrazole-3-carboxylic acid

or a pharmaceutically acceptable salt thereof.

Pharmaceutically acceptable salts include those described by Berge, Bighley and Monkhouse, *J. Pharm. Sci.,* 1977, 66, 1-19. The term "pharmaceutically acceptable salts" refers to salts prepared from pharmaceutically acceptable bases including inorganic bases and organic bases. Salts derived from inorganic bases include aluminum, ammonium, calcium, copper, ferric, ferrous, lithium, magnesium, manganic salts, manganous, potassium, sodium, zinc, and the like. Salts derived from pharmaceutically acceptable organic bases include salts of primary, secondary, and tertiary amines; substituted amines including naturally occurring substituted amines; and cyclic amines. Particular pharmaceutically acceptable organic bases include arginine, betaine, caffeine, choline, N,N'-dibenzylethylenediamine, diethylamine, 2-diethylaminoethanol, 2-dimethylaminoethanol, ethanolamine, ethylenediamine, N-ethyl-morpholine, N-ethylpiperidine, glucamine, glucosamine, histidine, hydrabamine, isopropylamine, lysine, methylglucamine, morpholine, piperazine, piperidine, procaine, purines, theobromine, triethylamine, trimethylamine, tripropylamine, tris(hydroxymethyl)aminomethane (TRIS, trometamol) and the like. Salts may also be formed from basic ion exchange resins, for example polyamine resins. When the compound of the present invention is basic, salts may be prepared from pharmaceutically acceptable acids, including inorganic and organic acids. Such acids include acetic, benzenesulfonic, benzoic, camphorsulfonic, citric, ethanesulfonic, ethanedisulfonic, fumaric, gluconic, glutamic, hydrobromic, hydrochloric, isethionic, lactic, maleic, malic, mandelic, methanesulfonic, mucic, pamoic, pantothenic, phosphoric, propionic, succinic, sulfuric, tartaric, p-toluenesulfonic acid, and the like.

The compounds of formula (I) may be prepared in crystalline or non-crystalline form, and, if crystalline, may optionally be hydrated or solvated. This invention includes within its scope stoichiometric hydrates or solvates as well as compounds containing variable amounts of water and/or solvent.

In a further aspect, this invention provides processes outlined in Schemes 1 and 2 for the preparation of a compound of formula (I):

Scheme 1

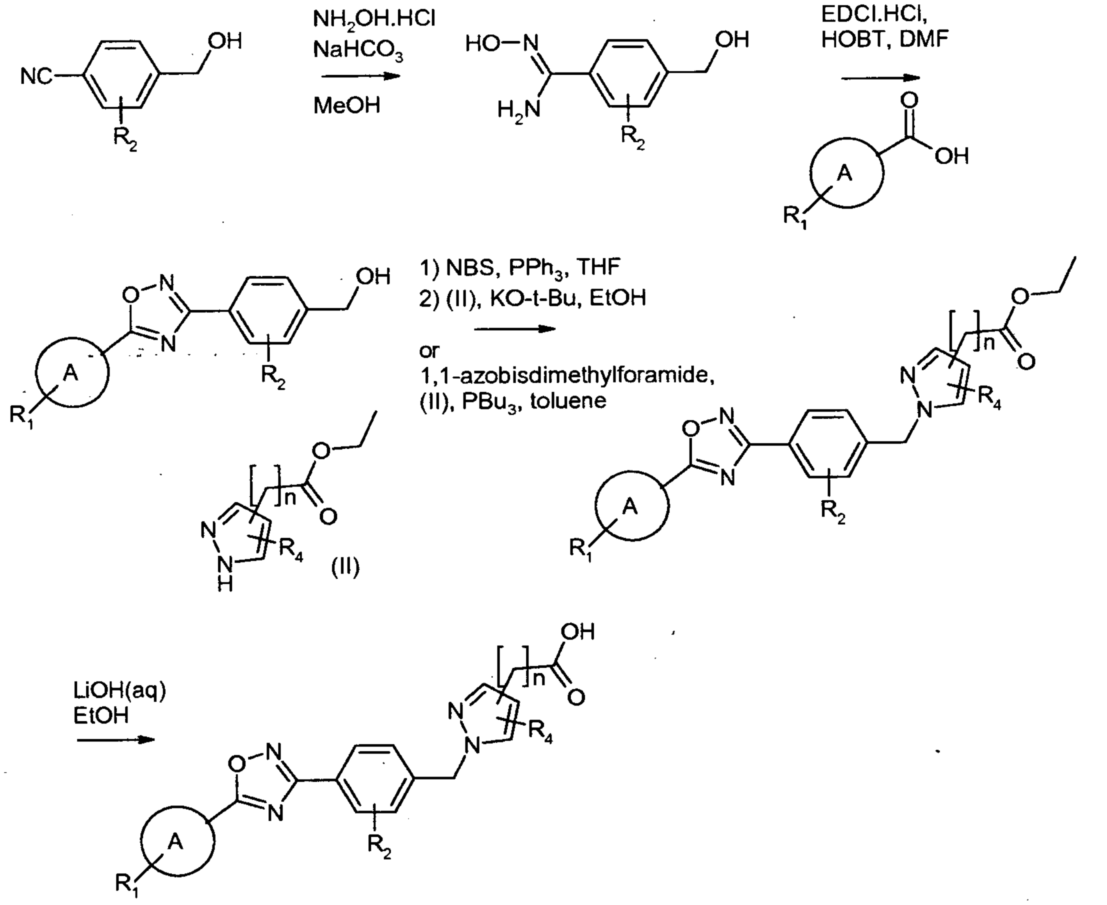
NH$_2$OH.HCl
NaHCO$_3$
MeOH
EDCI.HCl,
HOBT, DMF
1) NBS, PPh$_3$, THF
2) (II), KO-t-Bu, EtOH
or
1,1-azobisdimethylforamide,
(II), PBu$_3$, toluene
(II)
LiOH(aq)
EtOH

Scheme 2

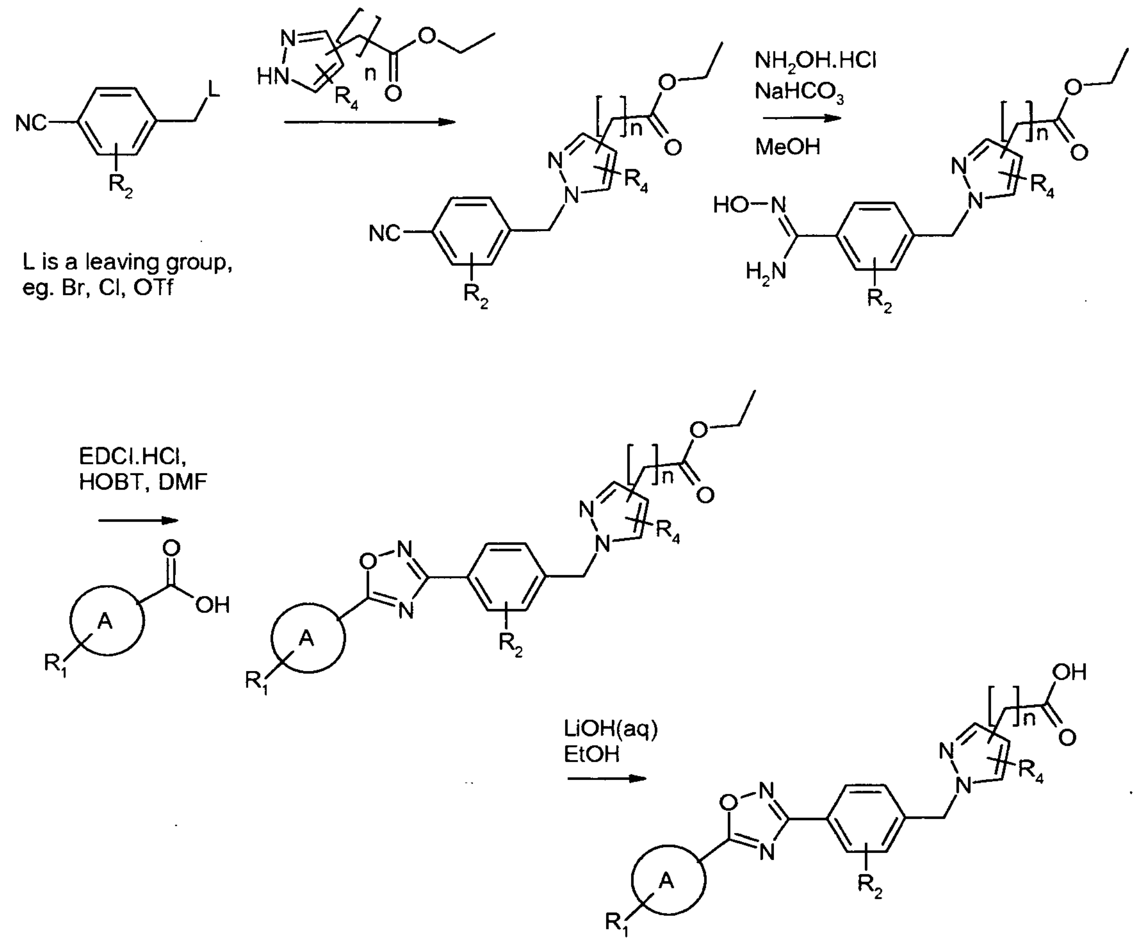


Pharmaceutically acceptable salts may be prepared conventionally by reaction with the appropriate acid or acid derivative.

The potencies and efficacies of the compounds of this invention for the S1 P1 receptor can be determined by GTPγS assay performed on the human cloned receptor as described herein or by the yeast binding assay, also described herein. Compounds of formula (I) have demonstrated agonist activity at the S1P1 receptor, using the functional assays described herein.

Compounds of formula (I) and their pharmaceutically acceptable salts are therefore of use in the treatment of conditions or disorders which are mediated via the S1 P1 receptor. In particular the compounds of formula (I) and their pharmaceutically acceptable salts are of use in the treatment of multiple sclerosis, autoimmune diseases, chronic inflammatory disorders, asthma, inflammatory neuropathies, arthritis, transplantation, Crohn's disease, ulcerative colitis, lupus erythematosis, psoriasis, ischemia-reperfusion injury, solid tumours, and tumour metastasis, diseases associated with angiogenesis, vascular diseases, pain conditions, acute viral diseases, inflammatory bowel conditions, insulin and non-insulin dependant diabetes (herein after referred to as the "Disorders of the Invention").

It is to be understood that "treatment" as used herein includes prophylaxis as well as alleviation of established symptoms.

Thus the invention also provides a compound of formula (I) or a pharmaceutically acceptable salt thereof, for use as a therapeutic substance, in particular in the treatment of the conditions or disorders mediated via the S1P1 receptor. In particular the invention provides a compound of formula (I) or a pharmaceutically acceptable salt thereof for use as a therapeutic substance in the treatment of multiple sclerosis, autoimmune diseases, chronic inflammatory disorders, asthma, inflammatory neuropathies, arthritis, transplantation, Crohn's disease, ulcerative colitis, lupus erythematosis, psoriasis, ischemia-reperfusion injury, solid tumours, and tumour metastasis, diseases associated with

angiogenesis, vascular diseases, pain conditions, acute viral diseases, inflammatory bowel conditions, insulin and non-insulin dependant diabetes. The invention further provides a method of treatment of conditions or disorders in mammals including humans which can be mediated via the S1 P1 receptor, which comprises administering to the sufferer a therapeutically safe and effective amount of a compound of formula (I) or a pharmaceutically acceptable salt thereof.

In another aspect, the invention provides for the use of a compound of formula (I) or a pharmaceutically acceptable salt thereof in the manufacture of a medicament for use in the treatment of the conditions or disorders mediated via the S1P1 receptor

In order to use the compounds of formula (I) in therapy, they will normally be formulated into a pharmaceutical composition in accordance with standard pharmaceutical practice. The present invention also provides a pharmaceutical composition, which comprises a compound of formula (I) or a pharmaceutically acceptable salt thereof, and a pharmaceutically acceptable carrier or excipient.

In a further aspect, the present invention provides a process for preparing a pharmaceutical composition, the process comprising mixing a compound of formula (I) or a pharmaceutically acceptable salt thereof and a pharmaceutically acceptable carrier or excipient.

A pharmaceutical composition of the invention, which may be prepared by admixture, suitably at ambient temperature and atmospheric pressure, is usually adapted for oral, parenteral or rectal administration and, as such, may be in the form of tablets, capsules, oral liquid preparations, powders, granules, lozenges, reconstitutable powders, injectable or infusible solutions or suspensions or suppositories. Orally administrable compositions are generally preferred.

Tablets and capsules for oral administration may be in unit dose form, and may contain conventional excipients, such as binding agents (e.g. pregelatinised maize starch, polyvinylpyrrolidone or hydroxypropyl methylcellulose); fillers (e.g. lactose, microcrystalline cellulose or calcium hydrogen phosphate); tabletting lubricants (e.g. magnesium stearate, talc or silica); disintegrants (e.g. potato starch or sodium starch glycollate); and acceptable wetting agents (e.g. sodium lauryl sulphate). The tablets may be coated according to methods well known in normal pharmaceutical practice.

Oral liquid preparations may be in the form of, for example, aqueous or oily suspension, solutions, emulsions, syrups or elixirs, or may be in the form of a dry product for reconstitution with water or other suitable vehicle before use. Such liquid preparations may contain conventional additives such as suspending agents (e.g. sorbitol syrup, cellulose derivatives or hydrogenated edible fats), emulsifying agents (e.g. lecithin or acacia), non-aqueous vehicles (which may include edible oils e.g. almond oil, oily esters, ethyl alcohol or fractionated vegetable oils), preservatives (e.g. methyl or propyl-p-hydroxybenzoates or sorbic acid), and, if desired, conventional flavourings or colorants, buffer salts and sweetening agents as appropriate. Preparations for oral administration may be suitably formulated to give controlled release of the active compound.

For parenteral administration, fluid unit dosage forms are prepared utilising a compound of formula (I) or pharmaceutically acceptable salt thereof and a sterile vehicle. Formulations for injection may be presented in unit dosage form e.g. in ampoules or in multi-dose, utilising a compound of formula (I) or pharmaceutically acceptable salt thereof and a sterile vehicle, optionally with an added preservative. The compositions may take such forms as suspensions, solutions or emulsions in oily or aqueous vehicles, and may contain formulatory agents such as suspending, stabilising and/or dispersing agents. Alternatively, the active ingredient may be in powder form for constitution with a suitable vehicle, e.g. sterile pyrogen-free water, before use. The compound, depending on the vehicle and concentration used, can be either suspended or dissolved in the vehicle. In preparing solutions, the compound can be dissolved for injection and filter sterilised before filling into a suitable vial or ampoule and sealing. Advantageously, adjuvants such as a local anaesthetic, preservatives and buffering agents are dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilisation cannot be accomplished by filtration. The compound can be sterilised by exposure to ethylene oxide before suspension in a sterile vehicle. Advantageously, a surfactant or wetting agent is included in the composition to facilitate uniform distribution of the compound.

Lotions may be formulated with an aqueous or oily base and will in general also contain one or more emulsifying agents, stabilising agents, dispersing agents, suspending agents, thickening agents, or colouring agents. Drops may be formulated with an aqueous or non-aqueous base also comprising one or more dispersing agents, stabilising agents, solubilising agents or suspending agents. They may also contain a preservative.

The compounds of formula (I) may also be formulated in rectal compositions such as suppositories or retention enemas, e.g. containing conventional suppository bases such as cocoa butter or other glycerides.

The compounds of formula (I) may also be formulated as depot preparations. Such long acting formulations may be administered by implantation (for example subcutaneously or intramuscularly) or by intramuscular injection. Thus, for example, the compounds of the invention may be formulated with suitable polymeric or hydrophobic materials (for example as an emulsion in an acceptable oil) or ion exchange resins, or as sparingly soluble derivatives, for example, as a sparingly soluble salt.

For intranasal administration, the compounds of formula (I) or a pharmaceutically acceptable salt thereof may be formulated as solutions for administration via a suitable metered or unitary dose device or alternatively as a powder mix with a suitable carrier for administration using a suitable delivery device. Thus compounds of formula (I) or pharmaceutically acceptable salts thereof may be formulated for oral, buccal, parenteral, topical (including ophthalmic and nasal), depot or rectal administration or in a form suitable for administration by inhalation or insufflation (either through the mouth or nose).

The compounds of the formula (I) or pharmaceutically acceptable salts thereof may be formulated for topical administration in the form of ointments, creams, gels, lotions, pessaries, aerosols or drops (e.g. eye, ear or nose drops). Ointments and creams may, for example, be formulated with an aqueous or oily base with the addition of suitable thickening and/or gelling agents. Ointments for administration to the eye may be manufactured in a sterile manner using sterilised components.

The composition may contain from 0.1% to 99% by weight, preferably from 10 to 60% by weight, of the active material, depending on the method of administration. The dose of the compound used in the treatment of the aforementioned disorders will vary in the usual way with the seriousness of the disorders, the weight of the sufferer, and other similar factors. However, as a general guide suitable unit doses may be 0.05 to 1000 mg, 1.0 to 500mg or 1.0 to 200 mg and such unit doses may be administered more than once a day, for example two or three times a day.

The compounds of the present invention may be used in combination preparations. For example, the compounds of the invention may be used in combination with cyclosporin A, methotrexate, steriods, rapamycin, proinflammatory cytokine inhibitors, other immunomodulators including antibodies or other therapeutically active compounds.

The subject invention also includes isotopically-labeled compounds, which are identical to those recited in formulas I and following, but for the fact that one or more atoms are replaced by an atom having an atomic mass or mass number different from the atomic mass or mass number usually found in nature. Examples of isotopes that can be incorporated into compounds of the invention include isotopes of hydrogen, carbon, nitrogen, oxygen, phosphorous, fluorine, iodine, and chlorine, such as $^{3}H$, $^{11}C$, $^{14}C$, $^{18}F$, $^{123}I$ and $^{125}I$.

Compounds of the present invention and pharmaceutically acceptable salts of said compounds that contain the aforementioned isotopes and/or other isotopes of other atoms are within the scope of the present invention. Isotopically-labeled compounds of the present invention, for example those into which radioactive isotopes such as $^{3}H$, $^{14}C$ are incorporated, are useful in drug and/or substrate tissue distribution assays. Tritiated, i.e., $^{3}H$, and carbon-14, i.e., $^{14}C$, isotopes are particularly preferred for their ease of preparation and detectability. $^{11}C$ and $^{8}F$ isotopes are particularly useful in PET (positron emission tomography), and $^{125}I$ isotopes are particularly useful in SPECT (single photon emission computerized tomography), all useful in brain imaging. Further, substitution with heavier isotopes such as deuterium, i.e., $^{2}H$, can afford certain therapeutic advantages resulting from greater metabolic stability, for example increased *in vivo* half-life or reduced dosage requirements and, hence, may be preferred in some circumstances. Isotopically labelled compounds of formula (I) and following of this invention can generally be prepared by carrying out the procedures disclosed in the Schemes and/or in the Examples below, by substituting a readily available isotopically labelled reagent for a non-isotopically labelled reagent.

The following Descriptions and Examples illustrate the preparation of compounds of the invention.

**<u>Conditions, Hardware and Software for Analytical LCMS Systems</u> Hardware**

Agilent 1100 Gradient Pump
Agilent 1100 Autosampler
Agilent 1100 DAD Dectector
Agilent 1100 Degasser
Agilent 1100 Oven
Agilent 1100 Controller
Waters Acquity Binary Solvent Manager
Waters Acquity Sample Manager
Waters Acquity PDA
Waters ZQ Mass Spectrometer
Sedere Sedex 55, Sedere Sedex 85, Sedere Sedex 75 or Polymer Labs PL-ELS-2100

**Software**

Waters MassLynx version 4.0 SP2 or version 4.1

**For 5 minute method**

**Column**

The column used is a Waters Atlantis, the dimensions of which are 4.6mm x 50mm. The stationary phase particle size is 3μm.

**Solvents**

A : Aqueous solvent = Water + 0.05% Formic Acid
B : Organic solvent = Acetonitrile + 0.05% Formic Acid

**Method**

The generic method used has a 5 minute runtime.

| Time/min | %B |
|---|---|
| 0 | 3 |
| 0.1 | 3 |
| 4 | 97 |
| 4.8 | 97 |
| 4.9 | 3 |
| 5.0 | 3 |

**Flow rate**

The above method has a flow rate of 3ml/mins

**For 2 minute method**

**Software**

Waters MassLynx version 4.1

**Column**

The column used is a Waters Acquity BEH UPLC C18, the dimensions of which are 2.1 mm x 50mm. The stationary phase particle size is 1.7μ m.

**Solvents**

A : Aqueous solvent = Water + 0.05% Formic Acid
B : Organic solvent = Acetonitrile + 0.05% Formic Acid
Weak Wash = 1:1 Methanol : Water
Strong Wash = Water

**Method**

The generic method used has a 2 minute runtime.

| Time / min | %B |
|---|---|
| 0 | 3 |
| 0.1 | 3 |
| 1.5 | 97 |
| 1.9 | 97 |
| 2.0 | 3 |

The above method has a flow rate of 1ml/min.
The injection volume for the generic method is 0.5ul
The column temperature is 40deg
The UV detection range is from 220 to 330nm

**Open Access Mass Directed Auto Prep System (MDAP)**

**Hardware**

Open Access Mass Directed Prep instruments consist of the following:

1 Waters 600 Gradient pump
1 Waters 2767 inject / collector
1 Waters Reagent manager
1 MicroMass ZQ Mass Spectrometer
1 Gilson Aspec - waste collector
1 Gilson 115 post-fraction UV detector
1 Computer System.

**Software**

MicroMass MassLynx v4.0

Column

The column used is typically a Supelco LCABZ++ column whose dimensions are 20mm internal diameter by 100mm in length. The stationary phase particle size is 5μm.

**Solvents**

A:. Aqueous solvent = Water + 0.1% Formic Acid
B:. Organic solvent = MeCN: Water 95:5 +0.05% Formic Acid
Make up solvent = MeOH: Water 80:20 +50mMol Ammonium Acetate
Needle rinse solvent = MeOH: Water: DMSO 80:10:10

**Methods**

One of five methods may be used depending on the analytical retention time of the compound of interest.
All have a 15-minute runtime, which comprises of a 10-minute gradient followed by a 5-minute column flush and re-equilibration step.

MDP 1.5-2.2 = 0-30% B

MDP 2.0-2.8 = 5-30% B
MDP 2.5-3.0 = 15-55% B
MDP 2.8-4.0 = 30-80% B
MDP 3.8-5.5 = 50-90% B

**Flow Rate**

All of the above methods have a flow rate of 20ml/min.

**Alternative system:,**

Hardware

- Waters 2525 Binary Gradient Module
- Waters 515 Makeup Pump
- Waters Pump Control Module
- Waters 2767 Inject Collect
- Waters Column Fluidics Manager
- Waters 2996 Photodiode Array Detector
- Waters ZQ Mass Spectrometer
- Gilson 202 fraction collector
- Gilson Aspec waste collector

Software

Waters MassLynx version 4 SP2

Column

The columns used are Waters Atlantis, the dimensions of which are 19mm x 100mm (small scale) and 30mm x 100mm (large scale). The stationary phase particle size is 5m m.

Solvents

A : Aqueous solvent = Water + 0.1% Formic Acid
B : Organic solvent = Acetonitrile + 0.1% Formic Acid
Make up solvent = Methanol : Water 80:20
Needle rinse solvent = Methanol

Methods

There are five methods used depending on the analytical retention time of the compound of interest. They have a 13.5-minute runtime, which comprises of a 10-minute gradient followed by a 3.5 minute column flush and re-equilibration step.

Large/Small Scale 1.0-1.5 = 5-30% B

Large/Small Scale 1.5-2.2 = 15-55% B

Large/Small Scale 2.2-2.9 = 30-85% B

Large/Small Scale 2.9-3.6 = 50-99% B

Large/Small Scale 3.6-5.0 = 80-99% B (in 6 minutes followed by 7.5 minutes flush and re-equilibration)

Flow rate

All of the above methods have a flow rate of either 20mls/min (Small Scale) or 40mls/min (Large Scale).

Shallow gradients

Large 1.5 to 2.3 min = 13-29% B

Large 1.9 to 2.3 min = 25-41% B

Large 2.3 to 2.6 min = 37-53% B

Large 2.6 to 3.1 min = 49-65% B

Large 3.1 to 3.6 min = 61-77% B

**Conditions used for NMR**

**Hardware**

Bruker 400MHz Ultrashield
Bruker B-ACS60 Autosampler
Bruker Advance 400 Console
Bruker DPX250
Bruker AVANCE 500
Bruker DRX600

**Software**

User interface - N MR Kiosk
Controlling software - XWin NMR version 3.0

**Chromatography**

Unless stated otherwise, all chromatography was carried out using silica columns

**Abbreviations:**

| | |
|---|---|
| g - | grams |
| mg - | milligrams |
| ml - | millilitres |
| ul - | microlitres |
| MeCN - | acetonitrile |
| MeOH - | methanol |
| EtOH - | ethanol |
| $Et_2O$ - | diethyl ether |
| EtOAc - | ethyl acetate |
| DCM - | dichloromethane |
| DIAD - | diisopropyl azodicarboxylate |
| DME - | 1,2-bis(methyloxy)ethane |
| DMF - | *N,N*-dimethylformamide |

| | |
|---|---|
| DMSO - | dimethylsulphoxide |
| EDAC - | *N*-(3-Dimethylaminopropyl)-*N'*-ethylcarbodiimide hydrochloride |
| EDC - | *N*-(3-Dimethylaminopropyl)-*N'*-ethylcarbodiimide hydrochloride |
| EDCI - | *N*-(3-Dimethylaminopropyl)-*N'*-ethylcarbodiimide hydrochloride |
| HOBT/HOBt - | Hydroxybenzotriazole |
| IPA - | isopropylalcohol |
| NCS - | N-chlorosuccinimide |
| PyBOP - | Benzotriazol-1-yl-oxytripyrrolidinophosphonium hexafluorophosphate |
| THF - | tetrahydrofuran |
| dba - | dibenzylidene acetone |
| RT - | room temperature |
| ˚C - | degrees Celsius |
| M - | Molar |
| H - | proton |
| s - | singlet |
| d - | doublet |
| t - | triplet |
| q - | quartet |
| MHz - | megahertz |
| MeOD - | deuterated methanol |
| LCMS - | Liquid Chromatography Mass Spectrometry |
| LC/MS - | Liquid Chromatography Mass Spectrometry |
| MS - | mass spectrometry |
| ES - | Electrospray |
| $MH^+$ - | mass ion + $H^+$ |
| MDAP - | mass directed automated preparative liquid chromatography. |
| sat. - | saturated |

**General chemistry section**

The intermediates for the preparation of the examples may not necessarily have been prepared from the specific batch described.

**Description 1**

**Ethyl 1H-pyrazole-3-carboxylate (D1)**

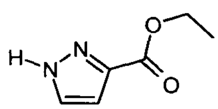

1H-pyrazole-3-carboxylic acid (1.0g, 8.9 mmol), was dissolved in ethanol (~12ml) and sulphuric acid added (1.5ml). the solution was heated at reflux overnight. The reaction mixture was concentrated *in vacuo* and water (~20ml) added, the solution was neutralised with sodium bicarbonate. Ethylacetate (~70ml) was added and the layers partitioned, the aq was extracted with ethylacetate (~70ml) and the combined organics were dried ($MgSO_4$) and concentrated *in vacuo* to yield the title compound as a white solid (1.21g, 8.64 mmol). δH ($CDCl_3$, 400 MHz): 7.77 (1H, d), 6.86 (1H, d), 4.43 (2H, quart), 1.42 (3H, t).

**Description 2**

**Ethyl 1-[(4-{5-[4-phenyl-5-(trifluoromethyl)-2-thienyl]-1,2,4-oxadiazol-3-yl}phenyl)methyl]-1*H*-pyrazole-4-carboxylate (D2)**

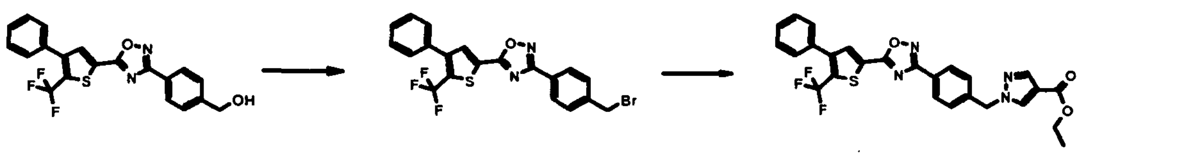

A mixture of (4-{5-[4-phenyl-5-(trifluoromethyl)-2-thienyl]-1,2,4-oxadiazol-3-yl}phenyl)methanol (WO 2003061567 A2) (103mg, 0.256mmol, 1.00 equivalents), triphenylphosphine (81mg, 0.307mmol, 1.20 equivalents) in tetrahydrofuran (1.2ml) was stirred at 0°C. N-bromosuccinimide (55mg, 0.307mmol, 1.05 equivalents) was added portion wise. Stirring was continued and the reaction was allowed to warm to room temperature. The reaction was monitored by LCMS. After 45 minutes the reaction mixture was partitioned between water (20ml) and ethyl acetate (45ml). The organic phase was separated, washed with brine (20ml x 2), dried with magnesium sulphate, filtered and evaporated to dryness. This produced 200mg of colourless oil (approximately 57% 3-[4-(bromomethyl)phenyl]-5-[4-phenyl-5-(trifluoromethyl)-2-thienyl]-1,2,4-oxadiazole), which was carried through to the next step without further purification.
MS: m/z $(M+H)^+$ 465, $C_{20}H_{12}BrF_3N_2OS$ requires 464 & 466.

A mixture of 3-[4-(bromomethyl)phenyl]-5-[4-phenyl-5-(trifluoromethyl)-2-thienyl]-1,2,4-oxadiazole (200mg, crude), ethyl 4-pyrazolecarboxylate (35mg, 0.25mmol), potassium *tert*-butoxide (31mg, 0.275mmol) in ethanol (2.5ml) was stirred at 60°C. The reaction was monitored by LCMS. After 16 hours the reaction mixture was evaporated to dryness, the residues were partitioned between ethyl acetate (45ml) and water (20ml). The organic phase was separated, washed with water (20ml) then brine (20ml), dried with magnesium sulphate, filtered and evaporated to dryness. This produced 287mg of yellow oil, which was purified on silica, eluting with pentane and ethyl acetate (0 to 90% gradient). The appropriate fractions were combined and evaporated to dryness, producing 186mg of white solid. The white solid was purified on silica, eluting with hexane and ethyl acetate (0 to 50% gradient). The appropriate fractions were combined and evaporated to dryness, producing 85mg of white solid (approximately 80%), which was carried through to the next step without further purification.
MS: m/z $(M+H)^+$ 525, $C_{26}H_{19}F_3N_4O_3S$ requires 524.

## Description 3

**Ethyl 1-[(4-{5-[4-phenyl-5-(trifluoromethyl)-2-thienyl]-1,2,4-oxadiazol-3-yl}phenyl)methyl]-1*H*-pyrazole-5-carboxylate (D3a) & Ethyl 1-[(4-{5-[4-phenyl-5-(trifluoromethyl)-2-thienyl]-1,2,4-oxadiazol-3-yl}phenyl)methyl]-1*H*-pyrazole-3-**carboxylate (D3b)

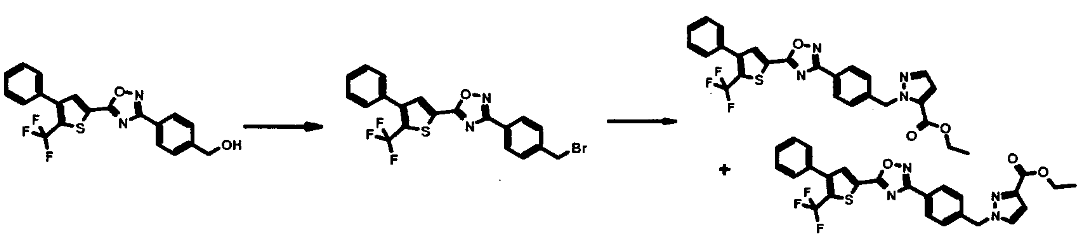

A mixture of (4-{5-[4-phenyl-5-(trifluoromethyl)-2-thienyl]-1,2,4-oxadiazol-3-yl}phenyl)methanol (WO 2003061567 A2) (134mg, 0.333mmol, 1.00 equivalents), triphenylphosphine (105mg, 0.400mmol, 1.20 equivalents) in tetrahydrofuran (1.7ml) was stirred at 0°C. N-bromosuccinimide (62mg, 0.350mmol, 1.05 equivalents) was added portion wise. Stirring was continued and the reaction was allowed to warm to room temperature. The reaction was monitored by LCMS. After 1 hour an additional 31 mg of N-bromosuccinimide was added and stirring continued. After 2 hours an additional 52mg of triphenylphosphine was added and stirring continued. After 2.5 hours the reaction mixture was partitioned between water (20ml) and ethyl acetate (45ml). The organic phase was separated, washed with brine (20ml x 2), dried with magnesium sulphate, filtered and evaporated to dryness. This produced 338mg of white solid (approximately 55% 3-[4-(bromomethyl)phenyl]-5-[4-phenyl-5-(trifluoromethyl)-2-thienyl]-1,2,4-oxadiazole), which was carried through to the next step without further purification.
MS: m/z $(M+H)^+$ 465 & 467, $C_{20}H_{12}BrF_3N_2OS$ requires 464 & 466.

A mixture of 3-[4-(bromomethyl)phenyl]-5-[4-phenyl-5-(trifluoromethyl)-2-thienyl]-1,2,4-oxadiazole (338mg, crude), ethyl 1*H*-pyrazole-3-carboxylate (47mg, 0.333mmol) and potassium *tert*-butoxide (41 mg, 0.366mmol) was stirred in ethanol (3.3ml) at 60°C. The reaction was monitored by LCMS. After 5 hours the reaction mixture was evaporated to dryness, the residues were partitioned between ethyl acetate (45ml) and water (20ml). The organic phase was separated, washed with brine (20ml), dried with magnesium sulphate, filtered and evaporated to dryness. This produced 300mg of orange oil, which was purified on silica, eluting with pentane and ethyl acetate (0 to 90% gradient). The appropriate fractions were combined and evaporated to dryness. Two isomers were isolated. **D3a** was obtained as a white solid (30mg, 17% over 2 steps).
$^1$H-NMR ($CDCl_3$): δ 1.34 (3H, t), 4.31 (2H, q), 5.85 (2H, s), 6.91 (1H, s), 7.36 (2H, d), 7.46 (5H, m), 7.58 (1H, s), 7.89 (1H, s), 8.08 (2H, d). MS: m/z $(M+H)^+$ 525, $C_{26}H_{19}F_3N_4O_3S$ requires 524.
63mg of yellow oil was also obtained, which was purified on a MDAP. The appropriate fractions were combined and evaporated to dryness, producing **D3b** as a white solid (48mg, 27% over 2 steps).
$^1$H-NMR ($CDCl_3$, 400MHz): δ 1.41 (3H, t), 4.43 (2H, q), 5.49 (2H, s), 6.87 (1H, s), 7.36 (2H, d), 7.41 (1H, s), 7.46 (5H, m), 7.90 (1H, s), 8.13 (2H, d). MS: m/z $(M+H)^+$ 525, $C_{26}H_{19}F_3N_4O_3S$ requires 524.

## Description 4

### Ethyl 1-[(4-{5-[4-phenyl-5-(trifluoromethyl)-2-thienyl]-1,2,4-oxadiazol-3-yl}phenyl)methyl]-1*H*-pyrazole-4-carboxylate (D4)

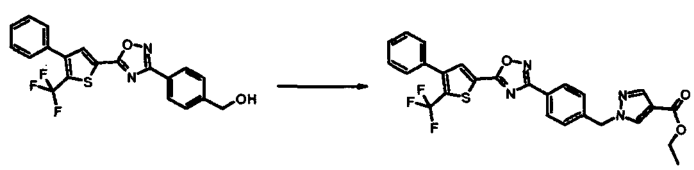

(4-{5-[4-Phenyl-5-(trifluoromethyl)-2-thienyl]-1,2,4-oxadiazol-3-yl}phenyl)methanol (WO 2003061567) (201mg, 0.500mmol, 1.00 equivalents), tributylphoshine (0.13ml, 0.525mmol, 1.05 equivalents) and ethyl 4-pyrazolecarboxylate (74mg, 0.525mmol, 1.05 equivalents) were suspended in dry toluene (4.0ml), 1,1-azobisdimethylformamide (90mg, 0.525mmol, 1.05 equivalents) was added and the reaction mixture stirred under argon. The reaction was monitored by LCMS. The reaction mixture initially forms a suspension, on the addition of 1,1-azobisdimethylformamide the mixture slowly dissolves, followed by the slow formation of a precipitate. After 16 hours the reaction mixture was diluted with ethyl acetate, filtered and evaporated to dryness, producing 467mg of cream a solid. The cream solid was purified on silica, eluting with pentane and ethyl acetate (0 to 90%). The appropriate fractions were combined and evaporated to dryness, producing the title compound as a white solid (210mg, 80%).
$^1$H-NMR ($CDCl_3$, 400MHz): δ 1.34 (3H, t), 4.29 (2H, q), 5.39 (2H, s), 7.38 (2H, d), 7.47 (5H, m), 7.91 (1H, s), 7.93 (1H, s), 7.98 (1H, s), 8.15 (2H, d). MS: m/z $(M+H)^+$ 525, $C_{26}H_{19}F_3N_4O_3S$ requires 524.

## Description 5

### 4-Bromo-5-(trifluoromethyl)-2-thiophenecarboxylic acid (D5)

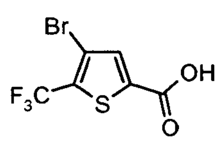

Diisopropylamine (2.1ml, 14.9mmol, 1.1 equivalents) in dry tetrahydrofuran (60ml) under argon was cooled to -65°C (internal temperature) and 2.5M *n*-butyllithium in hexanes (5.95ml, 14.9mmol, 1.1 equivalents) was added cautiously. The solution was allowed to warm to 0°C, after 20 minutes it was cooled to -40°C and treated slowly with 3-bromo-2-trifluoromethylthiophene (3.13g, 13.5mmol, 1 equivalent) in tetrahydrofuran (10ml). After stirring at -40°C for

20 minutes, the solution was allowed to warm to -10˚C for 5 minutes and then re-cooled to -40˚C. Carbon dioxide was bubbled through the reaction mixture and the reaction allowed to warm to +5˚C. The solvent was removed *in vacuo,* the residue treated with water (20ml) then acidified to pH1 by the addition of aqueous 2M hydrochloric acid. The mixture was extracted with 4 times ethyl acetate (70ml), and then combined, dried over sodium sulphate and concentrated *in vacuo* to give 4-bromo-5-(trifluoromethyl)-2-thiophenecarboxylic acid (2.69g) as a dark solid. $^1$H-NMR ($CDCl_3$, 400MHz): δ 7.80 (1H, q). MS: m/z $(M+H)^+$ 275, $C_6H_2BrF_3O_2S$ requires 275.

**Description 6**

**Ethyl 1-[(4-cyanophenyl)methyl]-1*H*-pyrazole-3-carboxylate (D6)**

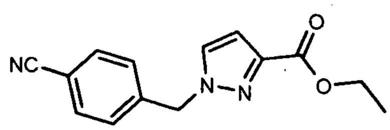

Benzyl bromide (12.46 g), ethyl 1*H*-pyrazole-3-carboxylate (D1) (8.89 g) and potassium *tert*-butoxide (8.75 g) were dissolved in EtOH (600 ml) and heated to 60 ˚C for 5 hours. The reaction mixture was then evaporated to dryness, re-dissolved in $H_2O$ and extracted with EtOAc (x 3). The combined organics were washed with brine, dried over $MgSO_4$ and evaporated to dryness. The crude residue was purified on a large Flash 75 cartridge, eluting with a 50 % mixture of EtOAc in hexane to give the desired isomer and then 75 % EtOAc in hexane to give the undesired isomer. The former was recrystallized from EtOAc/hexane to give the title compound (5.24 g) as white crystals. δH (MeOD, 400MHz): 1.41 (3H, t), 4.42 (2H, q), 5.47 (2H, s), 6.68 (1H, d), 7.28 (2H, d), 7.43 (1H, d), 7.64 (2H, d). MS (ES): $C_{14}H_{13}N_3O_2$ requires 255.28; found 256 (M+H).

**Description 7**

**Ethyl 1-({4-[(hydroxyamino)(imino)methyl]phenyl}methyl)-1*H*-pyrazole-3-carboxylate (D7)**

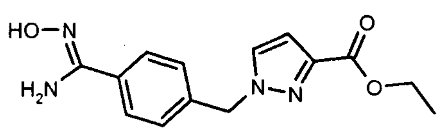

Ethyl 1-[(4-cyanophenyl)methyl]-1*H*-pyrazole-3-carboxylate (D6) (5.24 g), $NH_2OH$. HCl (2.85 g) and $NaHCO_3$ (8.62 g) were dissolved in EtOH (102.5 ml) and stirred at 50 ˚C overnight. The reaction mixture was then filtered, washing with EtOH then EtOAc. The filtrate was evaporated to give some product (0.5 g). The residue was further washed with MeOH to give the title compound (4.71 g) as an off white solid. δH (MeOD, 400MHz): 1.36 (3H, t), 4.35 (2H, q), 5.45 (2H, s), 6.81 (1H, d), 7.30 (2H, d), 7.63 (2H, d), 7.79 (1H, d). MS (ES): $C_{14}H_{16}N_4O_3$ requires 288.31; found 289 (M+H).

**Description 8**

**Ethyl 1-[(4-{5-[4-bromo-5-(trifluoromethyl)-2-thienyl]-1,2,4-oxadiazol-3-yl}phenyl)methyl]-1*H*-pyrazole-3-carboxylate (D8)**

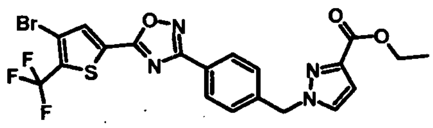

4-Bromo-5-(trifluoromethyl)-2-thiophenecarboxylic acid (D5) (200mg, 0.73mmol, 1 equivalent), *o*-benzotriazol-1-yl-N,N,N',N'-tetramethyluronium hexafluorophosphate (276mg, 0.73mmol, 1 equivalent), polymer bound 2-tert-butylimino-2-diethylamino-1,3-diemethyl-perhydro-1,3,2-diazaphosphorine (324mg, 0.73mmol, 1 equivalent) and acetonitrile (5ml) were stirred at room temperature for 10 minutes. Ethyl 1-({4-[(hydroxyamino)(imino)methyl]phenyl}methyl)-1*H*-pyrazole-3-carboxylate (D7) (230mg, 0.8mmol, 1.1 equivalents) and acetonitrile (1ml) were added and the reaction stirred for a further 1 hour. The reaction was then irradiated at 160˚C for 15 minutes using microwaves in a sealed vessel. The reaction mixture was filtered, washing through with methanol, then dichloromethane, and the filtrate concentrated *in vacuo.* The residue was taken up in ethyl acetate and water and washed with brine. The aqueous was extracted with 3 times ethyl acetate and the combined organics washed with brine, dried over sodium sulphate, filtered and concentrated *in vacuo* to yield a dark oil (439mg). This was triturated first with acetonitrile, secondly with dichloromethane and then thirdly with diethyl ether, followed by a dichloromethane wash. Each trituration yielded a small amount of solid which was combined to give an off-white solid (116mg). (approximately 96% of ethyl 1-[(4-{5-[4-bromo-5-(trifluoromethyl)-2-thienyl]-1,2,4-oxadiazol-3-yl}phenyl)methyl]-1*H*-pyrazole-3-carboxylate). The mother liquor was concentrated *in vacuo* and the residue (-300mg) was purified by column chromatography on silica (50g, 60ml) eluting 0-50% ethyl acetate/n-hexane over 15 column volumes. This yielded a brown solid (66mg) (approximately 74% of ethyl 1-[(4-{5-[4-bromo-5-(trifluoromethyl)-2-thienyl]-1,2,4-oxadiazol-3-yl}phenyl)methyl]-1*H*-pyrazole-3-carboxylate. Both batches of ethyl 1-[(4-{5-[4-bromo-5-(trifluoromethyl)-2-thienyl]-1,2,4-oxadiazol-3-yl}phenyl) methyl]-1*H*-pyrazole-3-carboxylate were dissolved in 1:1 methanol/dichloromethane and concentrated *in vacuo* to give an off-white solid (170mg) (approximately 91% ethyl 1-[(4-{5-[4-bromo-5-(trifluoromethyl)-2-thienyl]-1,2,4-oxadiazol-3-yl}phenyl) methyl]-1*H*-pyrazole-3-carboxylate).

$^1$H-NMR (DMSO, 400MHz): δ 5.56 (2H, s), 6.81 (1H, d), 7.44 (2H, d), 8.04-8.09 (3H, m), 8.39 (1H, d). MS: m/z $(M+H)^+$ 527, $C_{20}H_{14}{}^{79}BrF_3N_4O_3S$ requires 527.

**Description 9**

**1-Methylethyl 5-chloro-6-[(1-methylethyl)oxy]-3-pyridinecarboxylate (D9)**

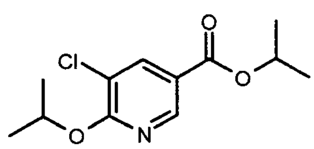

5-Chloro-6-hydroxy-3-pyridinecarboxylic acid (WO1997/02244)(1g, 5.76 mmol) was suspended in toluene (200ml) and treated with silver carbonate (3.97 g, 14.40 mmol) and 2-iodopropane (3.46 ml, 34.6 mmol) and stirred at RT in the dark for 3 days. LC/MS showed 2/3 product. Added 2-iodopropane (3ml) and stirred for 24 hours. LC/MS showed 80% product. Added EtOAc (200ml) and washed with water (200ml) + sat. NaHCO3 (50ml) followed by water (200ml). Dried over $MgSO_4$ and evaporated off the solvent to yield 1.0g of the title compound as a clear, colourless oil. MS $(ES^+)$ $C_{12}H_{16}{}^{35}ClNO_3$ requires 257; found 257.0.

**Description 10**

**5-Chloro-6-[(1-methylethyl)oxy]-3-pyridinecarboxylic acid (D10)**

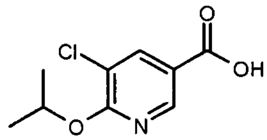

1-methylethyl 5-chloro-6-[(1-methylethyl)oxy]-3-pyridinecarboxylate (D9) (1.6 g, 6.21 mmol) in isopropanol (70 ml) and water (35.0 ml) was treated with 2N sodium hydroxide (6.21 ml, 12.42 mmol) and stirred for 3 hours to give a single product. Evaporated off the IPA, acidified with glacial acetic acid and extracted product into EtOAc (100ml). Dried over $MgSO_4$ and evaporated off the solvent to yield 1.30 g of the title compound as a white solid. MS (ES) $C_9H_{10}{}^{35}ClNO_3$ requires 215; found 214 (M-H$^+$).

**Description 11**

***N*-hydroxy-4-(hydroxymethyl)benzenecarboximidamide (D11)**

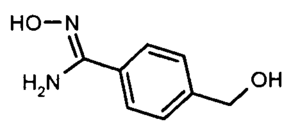

A mixture of 4-(hydroxymethyl)benzonitrile (998 mg), $NH_2OH$.HCl (1.07 g) and $NaHCO_3$ (3.15 g) in MeOH (15 ml) was stirred at 50 ˚C for 4.5 hours. The reaction mixture was then cooled to RT, neutralised with 1 M aqueous HCl and then extracted with EtOAc (2 x 30 ml). The EtOAc solutions were then washed with brine (20 ml), dried over $MgSO_4$, filtered and evaporated to dryness. This gave the title compound (875 mg) as a white solid. δH (DMSO, 400MHz): 4.50 (2H, d), 5.19 (1H, t), 5.76 (2H, br s), 7.30 (2H, d), 7.62 (2H, d), 9.55 (1H, s). MS (ES): $C_8H_{10}N_2O_2$ requires 166.18; found 167 (M+H).

Alternative procedure: A mixture of 4-(hydroxymethyl)benzonitrile (5.0 g), $NH_2OH$.HCl (5.3 g) and $NaHCO_3$ (12.6 g) in MeOH (100 ml) was refluxed for 6 hours. After cooling, the mixture was filtered and the filtrate evaporated to yield the title compound (7.0 g) as a white solid. δH (MeOH, 400MHz): 4.62 (2H, s), 7.39 (2H, d), 7.60 (2H, d).

**Description 12**

**3-chloro-4-[(1-methylethyl)oxy]benzoic acid (D12)**

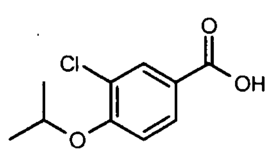

Methyl-4-hydroxy-3-chloro benzoate (13.4 g) was dissolved in DMF (150 ml), treated with $K_2CO_3$ (19.9 g) followed by isopropyl bromide (13.5 ml) and the resultant mixture heated to 70 ˚C and stirred overnight. The reaction mixture was then cooled to RT, evaporated to dryness, re-dissolved in EtOH, filtered and evaporated once more to give the intermediate ester (15.1g) as a white solid. This compound was a mixture of ethyl and methyl esters and used crude in the next reaction.

The crude intermediate (22.2 g) was dissolved in MeOH (75 ml), treated with 2M aqueous NaOH (75 ml), heated to 60 ˚C and stirred for 2 hours. The reaction mixture was then cooled to RT, the MeOH evaporated and the remaining aqueous solution acidified with 5M aqueous HCl (30 ml). The precipitate was filtered off and dried to give the title compound (15.1 g) as a white solid. δH ($CDCl_3$, 400MHz): 1.42 (6H, d), 4.70 (1H, septet), 6.97 (1H, d), 7.97 (1H,

d), 8.12 (1H, s). MS (ES): $C_{10}H_{11}ClO_3$ requires 214; found 213 (M-$H^+$).

**Description 13**

**[4-(5-{3-Chloro-4-[(1-methylethyl)oxy]phenyl}-1,2,4-oxadiazol-3-yl)phenyl]methanol (D13)**

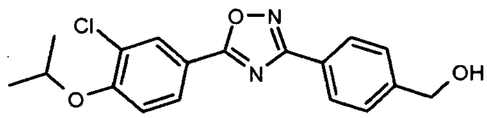

A mixture of 3-chloro-4-[(1-methylethyl)oxy]benzoic acid (D12) (558 mg), EDCI (498 mg), HOBT (352 mg) in DMF (3.3 ml) was stirred at room temperature for 20 minutes. N-hydroxy-4-(hydroxymethyl)benzenecarboximidamide (D11) (308 mg) was then added and the resulting slurry stirred at 100 ˚C for 4 hours. The reaction mixture was cooled to RT and evaporated to dryness. The residue was partitioned between EtOAc (75 ml) and $H_2O$ (40 ml). The organic phase was separated, washed with brine (40 ml), dried over $MgSO_4$, filtered and evaporated to dryness. The residue was purified on silica, eluting with a mixture of 10-100 % EtOAc in hexane. This gave the title compound (595 mg) as a yellow oil. δH ($CDCl_3$, 400MHz): 1.45 (6H, d), 4.72 (1H, quintet), 4.79 (2H, s), 7.06 (1H, d), 7.51 (2H, d), 8.06 (1H, dd), 8.15 (2H, d), 8.24 (1H, d). MS (ES): $C_{18}H_{17}ClN_2O_3$ requires 344.80; found 345 (M+H).

**Description 14**

**Ethyl 1-{[4-(5-{3-chloro-4-[(1-methylethyl)oxy]phenyl}-1,2,4-oxadiazol-3-yl)phenyl]methyl}-1*H*-pyrazole-3-carboxylate (D14)**

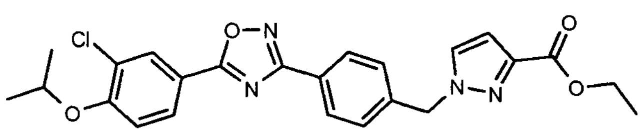

[4-(5-{3-Chloro-4-[(1-methylethyl)oxy]phenyl}-1,2,4-oxadiazol-3-yl)phenyl]methanol (D13) (200 mg) and ethyl 1*H*-pyrazole-3-carboxylate (D1) (85 mg) were dissolved in toluene (4.6 ml) under an Ar atmosphere and treated with tributylphosphine (152 μl). After stirring for 5 minutes 1,1'-azobis(N,N-dimethylformamide) (105 mg) was added and stirring continued overnight. The reaction mixture was then filtered, washing with toluene, and evaporated to dryness. The residue was purified by flash chromatography on silica, eluting with a 0-100 % EtOAc in pentane to give the undesired isomer (86 mg) and the title compound (108 mg) in poor purity. δH ($CDCl_3$, 400MHz): 1.42 (3H, t), 1.45 (6H, d), 4.43 (2H, q), 4.72 (1H, quintet), 5.48 (2H, s), 6.87 (1H, d), 7.06 (1H, d), 7.36 (2H, d), 7.41 (1H, d), 8.05 (1H, dd), 8.14 (2H, d), 8.23 (1H, d). MS (ES): $C_{24}H_{23}ClN_4O_4$ requires 466.92; found 467 (M+H).

**Description 15**

**4-(1-cyclohexen-1-yl)-3-(trifluoromethyl)benzamide (D15)**

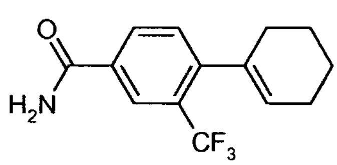

4-bromo-3-(trifluoromethyl)benzonitrile (1.2 g, 4.80 mmol), 1-cyclohexen-1-ylboronic acid (0.907 g, 7.20 mmol), sodium methoxide (0.778 g, 14.40 mmol) and bis(triphenylphosphine)palladium(II) chloride (0.337 g, 0.480 mmol) were added to dry methanol (12 mL) and the mixture heated in the microwave at 80 ˚C for 10 minutes. The reaction mixture was partitioned between ethyl acetate (40 mL) and water (40 mL) and then the organic phase washed with further water (40 mL). The organic phase was dried ($MgSO_4$), filtered and the solvent removed *in vacuo.* The crude product was purified by flash silica chromatography, eluting with 0-75 % ethyl acetate in hexane to give the title compound as a white solid (1.02 g). δH ($CDCl_3$, 400 MHz): 8.08 (1H, s), 7.90 (1H, d), 7.32 (1H, d), 6.3-5.8 (2H, m), 5.61 (1H, s), 2.25-2.13 (4H, m), 1.80-1.60 (4H, m) ppm. MS ($ES^+$): $C_{14}H_{14}F_3NO$ requires 269; found 270 ($M+H^+$).

## Description 16

**4-(1-cyclohexen-1-yl)-3-(trifluoromethyl)benzoic acid (D16)**

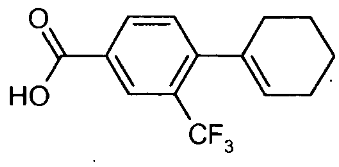

To a solution of 4-(1-cyclohexen-1-yl)-3-(trifluoromethyl)benzamide (D15) (100 mg, 0.371 mmol) in ethanol (3 ml) was added potassium hydroxide (208 mg, 3.71 mmol) and water (0.5 ml) and the reaction heated at 90 ˚C (block temperature) for 24 h. The reaction mixture was partitioned between ethyl acetate (20 mL) and aqueous HCl (2M, 15 mL) and then the aqueous phase extracted with further ethyl acetate (20 mL). The organic fractions were combined, dried (phase separator) and concentrated *in vacuo* to givethe title compound as a white solid (96 mg, 0.36 mmol). MS ($ES^-$): $C_{14}H_{13}F_3O_2$ requires 270; found 269 ($M-H^+$).

## Description 17

**4-cyclohexyl-3-(trifluoromethyl)benzamide (D17)**

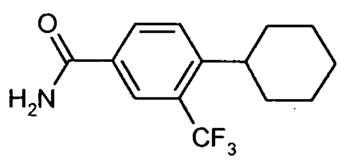

4-(1-cyclohexen-1-yl)-3-(trifluoromethyl)benzamide (D16) (850 mg, 3.16 mmol) was dissolved in methanol (63 ml) and hydrogenated using an H-Cube, using palladium on carbon at 40 ˚C with a flow rate of 2 mL/min. The solvent was removed *in vacuo* to give the title compound as a white solid (822 mg). δH ($CDCl_3$, 400 MHz): 8.07 (1H, s), 7.94 (1H, d), 7.53 (1H, d), 6.54 (2H, brs), 2.97 (1H, m), 1.90-1.75 (5H, m), 1.50-1.22 (5H, m) ppm. MS ($ES^+$): $C_{14}H_{16}F_3NO$ requires 271; found 272 ($M+H^+$).

## Description 18

**4-cyclohexyl-3-(trifluoromethyl)benzoic acid (D18)**

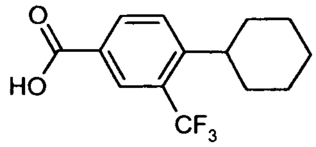

To a solution of 4-cyclohexyl-3-(trifluoromethyl)benzamide (D17) (822 mg, 3.03 mmol) in ethanol (40 ml) was added potassium hydroxide (1.700 g, 30.3 mmol) and water (5 ml) and the reaction heated to 90 °C block temperature for 3 h and stirred at room temperature for 16 h. Further potassium hydroxide (1.700 g, 30.3 mmol) was added and the reaction heated at reflux for 27 h. A further 5 mL of water was added and the reaction heated for 66 hours (weekend). The reaction mixture was concentrated *in vacuo* and the residue partitioned between ethyl acetate (25 mL) and aqueous hydrochloric acid (2M, 25 mL). The aqueous layer was further extracted with ethyl acetate (25 mL) and the combined organic phases dried ($MgSO_4$), filtered and the solvent removed *in vacuo* to give the title compound as a white solid (737 mg). δH (MeOD, 400 MHz): 8.24 (1H, s), 8.18 (1H, d), 7.68 (1H, d), 2.98 (1H, t), 1.89 (2H, m), 1.85-1.75 (3H, m), 1.58 (2H, dd), 1.50-1.30 (3H, m) ppm. MS ($ES^-$): $C_{14}H_{15}F_3O_2$ requires 272; found 271 ($M-H^+$).

**Description 19**

**1-methylethyl 4-[(1-methylethyl)oxy]-3-(trifluoromethyl)benzoate (D19)**

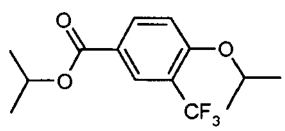

A mixture of 4-hydroxy-3-(trifluoromethyl)benzoic acid (450 mg, 2.18 mmol), 2-iodopropane (435 μL, 4.36 mmol) and potassium carbonate (603 mg, 4.36 mmol) in N,N'-dimethylformamide (40 mL) was heated at 70 °C for 4 h before further 2-iodopropane (218 μL, 2.18 mmol) was added and the heating continued for 18 h. The inorganic solid was filtered off and rinsed with ethyl acetate. The filtrate was concentrated *in vacuo* and the residue partitioned between ethyl acetate (150 mL) and water (150 mL) containing some aqueous sodium hydroxide. The organic layer was dried (phase separator) and concentrated *in vacuo* to give the title compound as a yellow oil. MS ($ES^+$): $C_{14}H_{17}F_3O_3$ requires 290; found 291 ($M+H^+$).

**Description 20**

**4-[(1-methylethyl)oxy]-3-(trifluoromethyl)benzoic acid (D20)**

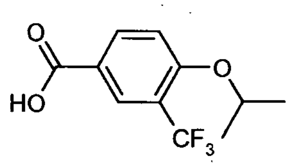

To a mixture of 1-methylethyl 4-[(1-methylethyl)oxy]-3-(trifluoromethyl)benzoate (D19) (704 mg, 2.43 mmol) in ethanol (110 mL) was added aqueous sodium hydroxide (2M, 12.2 mL, 24.3 mmol) and the reaction heated to reflux for 1 h. The mixture was concentrated *in vacuo* and the residue partitioned between ethyl acetate (100 mL) and water (100 mL) and acidified with aqueous hydrochloric acid (2M, 13 mL). The aqueous layer was extracted further with ethyl acetate (100 mL) and the combined organic layers dried and concentrated *in vacuo* to give the title compound as a yellow solid (563 mg). δH (MeOD, 400 MHz): 8.21-8.17 (2H, m), 7.26 (1H, d), 4.84 (1H, septet), 1.38 (6H, d) ppm. MS ($ES^-$): $C_{11}H_{11}F_3O_3$ requires 248; found 247 ($M-H^+$).

**Description 21**

**4-(2-methylpropyl)-3-(trifluoromethyl)benzamide (D21)**

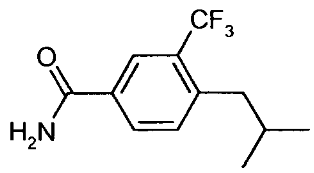

To a solution of 4-bromo-3-trifluoromethylbenzonitrile (1.25 g, 5.0 mmol) and isobutylzinc bromide (25 mmol) in THF (50 mL, 25 mmol) under argon was added 1,1'-bis(diphenylphosphino)ferrocenedichloro palladium(II) dichloromethane complex (612 mg, 0.75 mmol) and the reaction heated at reflux for 5 h. The mixture was concentrated *in vacuo* and the residue partitioned between ethyl acetate (80 mL) and water (80 mL). A solid formed and was filtered off and discarded. The organic layer was washed with water (80 mL) before it was dried (phase separator) and concentrated *in vacuo* to give the crude title compound as a black oil. This was used directly in the next step (1.35 g).

**Description 22**

**4-(2-methylpropyl)-3-(trifluoromethyl)benzoic acid (D22)**

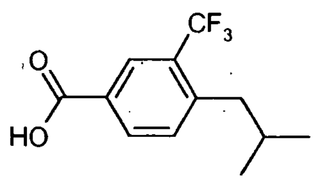

4-(2-methylpropyl)-3-(trifluoromethyl)benzamide (D21) (1.35 g, 5.50 mmol) was dissolved along with potassium hydroxide (3.09 g, 55.0 mmol) in ethanol (40 ml) and water (10.0 ml) and the solution heated to reflux for 18 h. The reaction mixture was concentrated *in vacuo* and the mixture separated between EtOAc (150 mL) and aqueous sodium hydroxide (2M, 150 mL). The layers were separated and the organic phase extracted with further sodium hydroxide solution (200 mL). LCMS of both phases showed product in both. Therefore the aqueous phase was acidified to pH1 with HCl (5M) and extracted back into EtOAc (2 x 150 mL) and these organic phases combined with the original organic phase. The solvent was removed *in vacuo* and the residue purified by reverse phase chromatography, eluting 5-100 % MeCN in $H_2O$ over 2000 mL and the solvent removed *in vacuo* to give a brown solid (690 mg, 2.410 mmol). This solid was triturated with hexane to give the title compound as a buff solid (135 mg, 0.548 mmol) and the filtrate purified by MDAP to give further title compound as a white solid (102 mg, 0.414 mmol). δH (DMSO, 400 MHz): 13.39 (1H, brs), 8.16 (1H, s), 8.13 (1H, d), 7.62 (1H, d), 2.69 (2H, d), 1.97 (1H, m), 0.90 (6H, d) ppm. LCMS ($ES^-$): $C_{12}H_{13}F_3O_2$ requires 246; found 245 ($M-H^+$); r.t. = 3.32 min (5 min method).

**Description 23**

**2-(Trifluoromethyl)-4-biphenylcarboxylic acid (D23)**

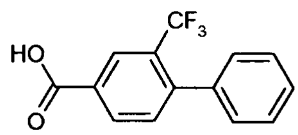

Batch A: A mixture of ethyl 4-chloro-3-trifluoromethylbenzoate (1.0 g, 3.96 mmol), phenyl boronic acid (724 mg, 5.94 mmol), palladium acetate (44 mg), (dicyclohexylphosphino)biphenyl (140.2 mg) and potassium fluoride (689 mg, 11.9 mmol) in THF (8 ml) was heated in the microwave at 120 ˚C for a total of 40 minutes. Batch B: A mixture of ethyl 4-chloro-3-trifluoromethylbenzoate (500 mg, 1.98 mmol), phenyl boronic acid (290 mg, 2.38 mmol), palladium acetate (2.2 mg), (dicyclohexylphosphino)biphenyl (7 mg) and potassium fluoride (344 mg, 5.8 mmol) in THF (4 ml) was heated in the microwave at 120 ˚C for 20 minutes.

The reaction mixtures from batches A & B were combined, filtered and the filtrate concentrated in vacuo. The residue was purified by flash chromatography ($SiO_2$, 0 to 5% EtOAc in hexane) to give a mixture of starting material and coupled product. This material was dissolved in ethanol (10 ml) and 2M NaOH (aq) (5ml) and then heated to reflux for 3 h. The solvent was removed in vacuo and the residue partitioned between DCM and 2M aq. HCl. The aq. was extracted with further DCM. The organic phases were combined and concentrated in vacuo. The crude material was purified by reverse phase chromatography eluting with 5 to 100% MeCN in water to afford the title compound as a white solid (367 mg, 1.38 mmol). δH (DMSO, 400 MHz) 7.31-7.40 (2H, m), 7.44-7.52 (3H, m), 7.57 (1H, d), 8.24 (1H, dd), 8.29 (1H, d), 13.57 (1H, br. s). LCMS ($ES^-$): $C_{14}H_9F_3O_2$ requires 266; found 265 ($M-H^+$);

**Description 24**

**2'-fluoro-2-(trifluoromethyl)-4-biphenylcarboxylic acid (D24)**

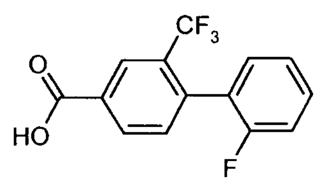

To 4-bromo-3-trifluoromethylbenzonitrile (1.5 g, 6.0 mmol) and 2-fluorophenylbenzoic acid (1.0 g, 7.2 mmol) under a flush of argon was added toluene (30 mL), saturated aqueous sodium carbonate (10 mL) and ethanol (10 mL) before addition of palladium tetrakistriphenylphosphine (345 mg, 0.3 mmol). The mixture was heated to 90 ˚C (block temperature) for 19 h. The reaction mixture was partitioned between ethyl acetate (100 ml) and water (80 mL). The organic phase was washed with brine (80 mL) before it was dried (phase separator) and concentrated *in vacuo.* The residue was purified by silica chromatography, eluting 0-75 % EtOAc in hexane. The resulting product was then dissolved in ethanol (40 mL) and water (10 mL) and potassium hydroxide (2.5 g, 44 mmol) was added. The solution was heated overnight to 90 ˚C (block temperature). The reaction mixture was concentrated and partitioned between ethyl acetate (70 mL) and 2M HCl (30 mL). The organic phase was washed with further 2M HCl (30 mL) before it was dried (phase separator) and the solvent removed *in vacuo.* The crude product was purified by reverse phase chromatography, eluting 5-100 % acetonitrile in water to give the title compound as a solid (551 mg, 1.94 mmol). MS ($ES^-$): $C_{14}H_8F_4O_2$ requires 284; found 283 ($M-H^+$).

**Description 25**

**4-bromo-3-ethylbenzonitrile (D25)**

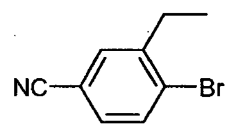

A mixture of 4-amino-3-ethylbenzonitrile (3.00 g, 20.5 mmol) and copper (II) bromide (6.88 g, 30.8 mmol) in acetonitrile (115 mL) was stirred under argon before addition of isoamylnitrite (3.60 g, 30.8 mmol) dropwise through a condenser and the mixture was heated to 60 ˚C for 2.5 h. The mixture was stood at room temperature overnight under argon before it was concentrated. Diethyl ether was added and the mixture washed with saturated aqueous ammonium chloride followed by 2M sodium hydroxide. The aqueous was extracted with further diethyl ether before the combined organic fractions were dried (phase separator) and evaporated. Purification of the material in three batches was by reverse phase chromatography, eluting 5-100 % acetonitrile in water to give the title compound 787 mg, 3.74 mmol). δH (MeOD, 400 MHz): 7.74 (1H, d), 7.67 (1H, d), 7.45 (1H, dd), 2.82 (2H, q), 1.24 (3H, t) ppm

**Description 26**

**2-ethyl-4-biphenylcarboxylic acid (D26)**

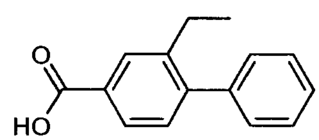

A mixture of 4-bromo-3-ethylbenzonitrile (D25) (270 mg, 1.30 mmol), phenylboronic acid (302 mg, 2.48 mmol), palladiumtetrakistriphenylphosphine (144 mg, 0.12 mmol) and potassium carbonate (514 mg, 3.72 mmol) in dimethylformamide (4 mL) was heated to 170 ˚C in the microwave for 30 minutes. The mixture was allowed to stand at room temperature overnight before it was poured into a mixture of ice and saturated aqueous ammonium chloride. The mixture was extracted with ethyl acetate (3 x 20 mL) and the combined organics washed with brine, dried (phase separator) and evaporated. The residue was purified by silica chromatography, eluting 0-8 % EtOAc in hexane to give a colourless oil (272 mg). This material was added to ethanol (12 mL) and water (3 mL) followed by potassium hydroxide (730 mg, 13.0) mmol and the mixture heated to 90 ˚C overnight. The solvent was evaporated and the residue partitioned between ethyl acetate (40 mL) and 2M HCl (15 mL). The organic layer was washed with further HCl (15 mL) before it was dried (phase separator) and concentrated *in vacuo.* The resulting solid was purified by MDAP and the product containing fractions concentrated *in vacuo.* The residue was partitioned between ethyl acetate and 2M HCl. The organic phase was washed with further 2M HCl, dried (phase separator) and evaporated to give the title compound as a white solid (166 mg, 0.73 mmol). MS ($ES^-$): $C_{15}H_{14}O_2$ requires 226; found 225 ($M-H^+$).

**Description 27**

**2-ethyl-2'-fluoro-4-biphenylcarboxylic acid (D27)**

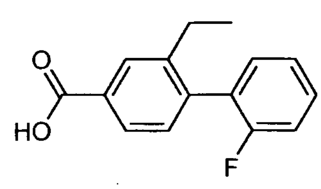

A mixture of 4-bromo-3-ethylbenzonitrile (D25) (651 mg, 3.10 mmol), 2-fluorophenylboronic acid (867 mg, 6.20 mmol), palladiumtetrakistriphenylphosphine (359 mg, 0.31 mmol) and potassium carbonate (1.29 g, 9.30 mmol) in

dimethylformamide (10 mL) was heated to 170 ˚C in the microwave for 30 minutes. The mixture was allowed to stand at room temperature over a weekend before it was poured into a mixture of ice and saturated aqueous ammonium chloride. The mixture was extracted with ethyl acetate (3 x 20 mL) and the combined organics washed with brine, dried (phase separator) and evaporated. The residue was purified by silica chromatography, eluting 0-5 % EtOAc in hexane to give a colourless oil (655 mg). This material was added to ethanol (26 mL) and water (6 mL) followed by potassium hydroxide (1.62 g, 28.9 mmol) and the mixture heated to 90 ˚C for 17 h. The solvent was evaporated and the residue partitioned between ethyl acetate and 2M HCl. The organic layer was washed with further HCl before it was dried (phase separator) and concentrated *in vacuo* to give the title compound as a white solid (648 mg, 2.66 mmol). MS ($ES^-$): $C_{15}H_{13}FO_2$ requires 244; found 243 ($M-H^+$).

**Description 28**

**Methyl 1-[(4-{5-[2'-fluoro-2-(trifluoromethyl)-4-biphenylyl]-1,2,4-oxadiazol-3-yl}phenyl)methyl]-1H-pyrazole-3-carboxylate (D28)**

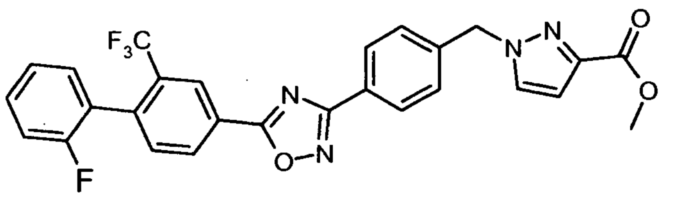

2'-fluoro-2-(trifluoromethyl)-4-biphenylcarboxylic acid (104 mg, 0.365 mmol), EDC (77 mg, 0.401 mmol) and HOBT (61.4 mg, 0.401 mmol) were dissolved in N,N-dimethylformamide (5 ml) and stirred at r.t. for 15 minutes. Methyl 1-({4-[(hydroxyamino)(imino)methyl]phenyl}methyl)-1H-pyrazole-3-carboxylate (D7) (100 mg, 0.365 mmol) was then added and the reaction stirred at r.t. for 1 h. The reaction was then heated at 80 ˚C for 18 h. The reaction mixture was partitioned between EtOAc (25 mL) and water (25 mL) and the organic phase washed with aqueous sodium bicarbonate (25 mL) and water (25 mL) before it was dried (phase separator) and concentrated *in vacuo.* The residue was triturated with ethanol to give a white solid, methyl 1-[(4-{5-[2'-fluoro-2-(trifluoromethyl)-4-biphenylyl]-1,2,4-oxadiazol-3-yl}phenyl) methyl]-1H-pyrazole-3-carboxylate (77 mg, 0.147 mmol). MS ($ES^+$): requires $C_{27}H_{18}F_4N_4O_3$ 522; found 523 ($M+H^+$).

**Descriptions 29-35**

The following compounds were made by a similar method: Variations included reaction time and purification method. The trituration step may have been performed with 1:1 MeOH:DMSO. For some examples, after trituration the filtrate was purified by reverse phase chromatography to give further material, or both solid and filtrate were combined and purified by reverse phase chromatography.

| | Structure | Name | MS ($ES^+$) |
|---|---|---|---|
| D29 | 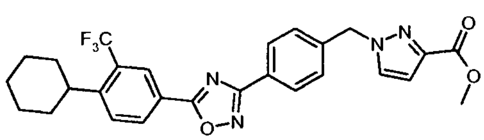 | methyl 1-[(4-{5-[4-cyclohexyl-3-(trifluoromethyl)phenyl]-1,2,4-oxadiazol-3-yl}phenyl)methyl]-1*H*-pyrazole-3-carboxylate | 511 |
| D30 | 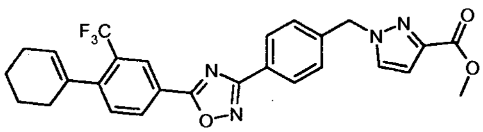 | methyl 1-[(4-{5-[4-(1-cyclohexen-1-yl)-3-(trifluoromethyl)phenyl]-1,2,4-oxadiazol-3-yl}phenyl)methyl]-1*H*-pyrazole-3-carboxylate | 509 |

(continued)

| | Structure | Name | MS (ES+) |
|---|---|---|---|
| D31 | 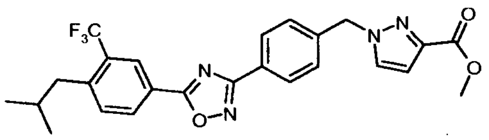 | methyl 1-[(4-{5-[4-(2-methylpropyl)-3-(trifluoromethyl)phenyl]-1,2,4-oxadiazol-3-yl}phenyl)methyl]-1*H*-pyrazole-3-carboxylate | 485 |
| D32 | 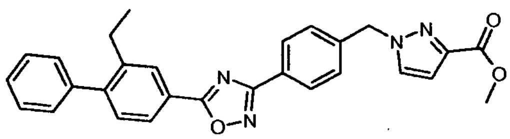 | methyl 1-({4-[5-(2-ethyl-4-biphenylyl)-1,2,4-oxadiazol-3-yl]phenyl}methyl)-1*H*-pyrazole-3-carboxylate | 465 |
| D33 | 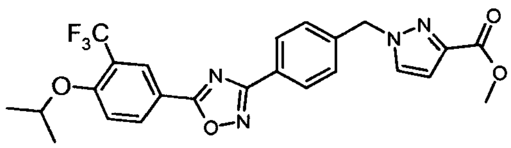 | methyl 1-[(4-{5-[4-[(1-methylethyl)oxy]-3-(trifluoromethyl)phenyl]-1,2,4-oxadiazol-3-yl}phenyl)methyl]-1*H*-pyrazole-3-carboxylate | 487 |
| D34 | 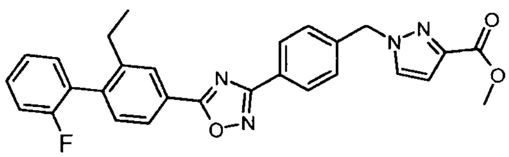 | methyl 1-({4-[5-(2-ethyl-2'-fluoro-4-biphenylyl)-1,2,4-oxadiazol-3-yl] phenyl}methyl)-1*H*-pyrazole-3-carboxylate | 483 |
| D35 | 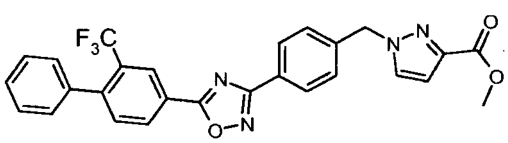 | methyl 1-[(4-{5-[2-(trifluoromethyl)-4-biphenylyl]-1,2,4-oxadiazol-3-yl} phenyl)methyl]-1*H*-pyrazole-3-carboxylate | 505 |

**Description 36**

**Ethyl 1-[(3-chloro-4-cyanophenyl)methyl]-1H-pyrazole-3-carboxylate (D36)**

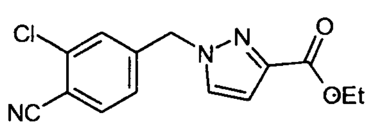

A suspension of ethyl 1H-pyrazole-3-carboxylate (D1) (280mg, 2.0 mmol), 4-(bromomethyl)-2-chlorobenzonitrile [WO 2007/039177] (460mg, 2 mmol) and potassium t-butoxide (246mg, 2.2 mmol), in ethanol (15ml) was heated at 60°C for 3 hours. The reaction mixture was concentrated in vacuo and dissolved in water (~25ml) and ethylacetate (~25ml), the layers were partitioned and the aq was washed twice with ethylacetate (2x 15ml). The combined organics were passed through a phase separating cartridge to dry and concentrated in vacuo. The crude product was purified by Biotage SP4 with 25S cartridge and 10-70% ethylacetate/hexane as eluant. The product containing fractions were combined to generate two products, one of which was the title compound as a white solid (211 mg, 0.73 mmol). MS

(ES+): requires $C_{14}H_{12}{}^{35}ClN_3O_2$ 289; found 290 (M+H+).

**Description 37**

**Ethyl 1-({3-chloro-4-[(Z)-(hydroxyamino)(imino)methyl]phenyl}methyl)-1H-pyrazole-3-carboxylate (D37)**

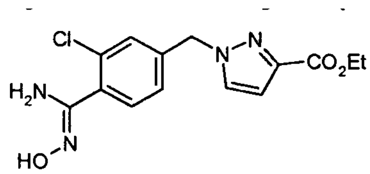

A suspension of ethyl 1-[(3-chloro-4-cyanophenyl)methyl]-1H-pyrazole-3-carboxylate (D36) (211mg, 0.73 mmol), in ethanol (5ml), with hydrozylamine hydrochloride (101mg, 1.45 mmol), and sodium bicarbonate (306mg, 3.64 mmol), was heated over the weekend at 50˚C. The reaction mixture was allowed to cool and ethanol (~15ml) was added to the mixture, this was stirred for ~5minutes and the mixture was filtered. The resultant solution was concentrated in vacuo to yield the title compound as a white solid (256mg, 0.80 mmol). MS (ES+): requires $C_{14}H_{15}{}^{35}ClN_4O_3$ 322; found 323 (M+H+).

**Description 38**

**Ethyl 1-[(3-chloro-4-{5-[4-phenyl-5-(trifluoromethyl)-2-thienyl]-1,2,4-oxadiazol-3-yl}phenyl)methyl]-1H-pyrazole-3-carboxylate (D38)**

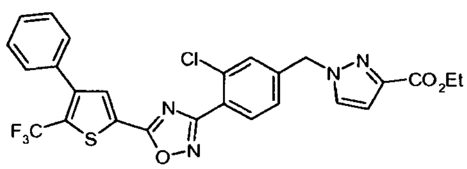

Ethyl 1-({3-chloro-4-[(Z)-(hydroxyamino)(imino)methyl]phenyl}methyl)-1H-pyrazole-3-carboxylate (D37) (256mg, 0.80 mmol), 4-phenyl-5-(trifluoromethyl)-2-thiophenecarboxylic acid (432mg, 1.58 mmol), HOBt (118mg, 0.87 mmol), and EDAC (167mg, 0.87 mmol), were heated in DMF (10ml) at 80˚C over night. The reaction mixture was allowed to cool, further HOBt (118mg, 0.87 mmol), EDAC (167mg, 0.874 mmol), and 4-phenyl-5-(trifluoromethyl)-2-thiophenecarboxylic acid (432mg, 1.58 mmol), was added to the reaction mixture and the mixture was heated at 80˚C over night. The reaction mixture was concentrated in vacuo and dissolved in ethylacetate (~50ml) and water (~50ml). The layers were partitioned and separated, the aq layer extracted with ethylacetate (2x ~30ml). The combined organics were concentrated in vacuo and dissolved in methanol (minimum volume). The solution was purified by Biotage Horizon reverse phase $C_{18}$ 25S cartridge with 5-100% acetonitrile/water as eluant. The product containing fractions were concentrated *in vacuo* to yield the title compound as a white solid (54mg, 0.01 mmol). MS (ES+): requires $C_{26}H_{18-}{}^{35}ClF_3N_4O_3S$ 558; found 559 (M+H+).

**Description 39**

**Ethyl 1-[(2-chloro-4-cyanophenyl)methyl]-1H-pyrazole-3-carboxylate (D39)**

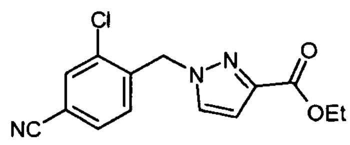

The title compound was prepared by a method similar to D36, using 4-(bromomethyl)-3-chlorobenzonitrile in place of 4-(bromomethyl)-2-chlorobenzonitrile, to yield the title compound as a white solid (120mg, 0.415 mmol). MS ($ES^+$): $C_{14}H_{12-}$ $^{35}ClN_3O_2$ requires 289; found 290 ($M+H^+$).

**Description 40**

**Ethyl 1-({2-chloro-4-[(Z)-(hydroxyamino)(imino)methyl]phenyl}methyl)-1H-pyrazole-3-carboxylate (D40)**

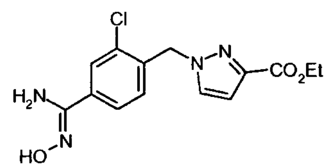

The title compound was prepared by a method similar to D37, using ethyl 1-[(2-chloro-4-cyanophenyl)methyl]-1H-pyrazole-3-carboxylate (D39) in place of ethyl 1-[(3-chloro-4-cyanophenyl)methyl]-1H-pyrazole-3-carboxylate (D36), to yield a white solid (200mg, 0.62 mmol). MS ($ES^+$): requires $C_{14}H_{15}{}^{35}ClN_4O_3$ 322; found 323 ($M+H^+$).

**Description 41**

**Ethyl 1-[(2-chloro-4-{5-[4-phenyl-5-(trifluoromethyl)-2-thienyl]-1,2,4-oxadiazol-3-yl}phenyl)methyl]-1H-pyrazole-3-carboxylate (D41)**

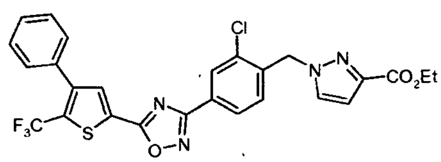

Ethyl 1-({2-chloro-4-[(Z)-(hydroxyamino)(imino)methyl]phenyl}methyl)-1H-pyrazole-3-carboxylate (D40) (200mg, 0.62 mmol), 4-phenyl-5-(trifluoromethyl)-2-thiophenecarboxylic acid (186mg, 0.68 mmol), HOBt (92mg, 0.68 mmol), and EDAC (131 mg, 0.69 mmol), were stirred in DMF (5ml) at room temperature for 40 hours. The reaction mixture was heated to 80˚C for 4 hours, heating was continued at 80˚C over night. The reaction mixture was allowed to cool and left to stand for 2 days. The reaction mixture was concentrated in vacuo and dissolved in ethylacetate (~50ml) and water (~50ml). The layers were partitioned and separated, the aq layer extracted with ethylacetate (3x ~20ml). The combined organics were concentrated in vacuo and dissolved in methanol (minimum volume). The solution was purified by Biotage Horizon reverse phase $C_{18}$ 25S cartridge with 5-100% acetonitrile/water as eluant. The product containing fractions were concentrated in vacuo to yield the title compound as a white solid (85mg, 0.15 mmol). MS ($ES^+$): requires $C_{26}H_{18-}$ $^{35}ClF_3N_4O_3S$ 558; found 559 ($M+H^+$).

**Description 42**

**2-Bromo-4-(bromomethyl)benzonitrile (D42)**

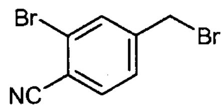

NBS (9.57g, 0.054mol), and benzoylperoxide (130mg, 0.54mmol), were added to a solution of 4-methyl-2-bromo benzonitrile (9.0g, 0.046mol), in benzene (300ml). The reaction mixture was heated at 80˚C for 2hours. The reaction mixture was left to stand over night, heating at 80˚C was resumed for 8 hours. The reaction mixture was diluted with ethyl acetate (~200ml) and water (~200ml), the layers were partitioned and the organic was passed through a phase separating cartridge and concentrated in vacuo. The residue was triturated with ether and the solid filtered to yield the title compound (6.48g, 0.024mol). δH (DMSO, 400 MHz): 8.00 (1H, d), 7.96 (1H, d), 7.67, (1H, dd), 4.75, (2H, s).

**Description 43**

**Ethyl 1-[(3-bromo-4-cyanophenyl)methyl]-1H-pyrazole-3-carboxylate (D43)**

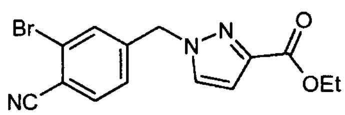

A suspension of ethyl 1H-pyrazole-3-carboxylate (D1) (3.298g, 0.024 mol), 4-(bromomethyl)-2-bromobenzonitrile (D42) (6.48g, 0.024 mol) and potassium t-butoxide (2.903g, 0.026 mol), in ethanol (250ml) was heated at 60˚C overnight. The reaction mixture was concentrated in vacuo and dissolved in water (~250ml) and ethylacetate (~250ml), the layers were partitioned and the aq was washed twice with ethylacetate (2x 100ml). The combined organics were passed through a phase separating cartridge to dry and concentrated in vacuo. The residue was scratched down and stirred in ethylacetate (~50ml) and the resultant solid was filtered off. The mother liquors were concentrated in vacuo and purified by Biotage SP4 with 40M cartridge and 30-70% ethylacetate/hexane as eluant. The product containing fractions were combined and concentrated. This product was combined with the first crop to yield the title compound as a white solid (1.55g, 4.6 mmol). MS ($ES^+$): requires $C_{14}H_{12}{}^{79}BrN_3O_2$ 333; found 334 ($M+H^+$).

**Description 44**

**Ethyl 1-({3-bromo-4-[(Z)-(hydroxyamino)(imino)methyl]phenyl}methyl)-1H-pyrazole-3-carboxylate (D44)**

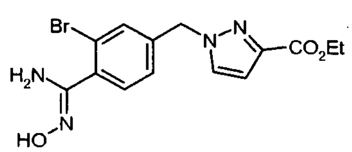

A suspension of ethyl 1-[(3-bromo-4-cyanophenyl)methyl]-1H-pyrazole-3-carboxylate (D43) (1.55g, 4.6 mmol), in ethanol (50ml), with hydroxylamine hydrochloride (645mg, 9.2 mmol), and sodium bicarbonate (1.95g, 0.023 mol), was heated over night at 50˚C. The reaction mixture was diluted with ethanol (300ml) and the solution heated at 50˚C for 3 hours. Hydroxylamine hydrochloride (1.29g, 18.4 mmol) was added and the reaction mixture heated at 50˚C for 3

hours. The reaction mixture was allowed to cool the mixture was filtered. The resultant solution was concentrated in vacuo to yield the title compound as a white solid (1.2g, 3.26 mmol). MS ($ES^+$): requires $C_{14}H_{15}{}^{79}BrN_4O_3$ 366; found 367 ($M+H^+$).

## Description 45

**Ethyl 1-{[3-bromo-4-(5-{3-cyano-4-[(1-methylethyl)oxy]phenyl}-1,2,4-oxadiazol-3-yl)phenyl]methyl}-1H-pyrazole-3-carboxylate (D45)**

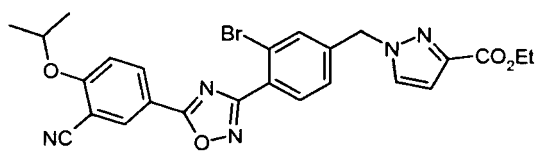

The title compound was prepared by a method similar to D38, using ethyl 1-({3-bromo-4-[(Z)-(hydroxyamino)(imino)methyl]phenyl}methyl)-1H-pyrazole-3-carboxylate (D44) and 3-Cyano-4-[(1-methylethyl)oxy]benzoic acid (WO2005/58848) in place of ethyl 1-({3-chloro-4-[(hydroxyamino)(imino) methyl] phenyl}methyl)-1H-pyrazole-3-carboxylate (D37) and 4-phenyl-5-(trifluoromethyl)-2-thiophene carboxylic acid, to yield the title compound as a white solid (506mg, 0.94 mmol). MS ($ES^+$): requires $C_{25}H_{22}{}^{79}BrN_5O_4$ 535; found 536 ($M+H^+$).

## Example 1

**1-[(4-{5-[4-Phenyl-5-(trifluoromethyl)-2-thienyl]-1,2,4-oxadiazol-3-yl}phenyl)methyl]-1*H*-pyrazole-4-carboxylic acid (E1)**

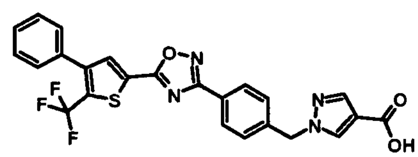

A mixture of ethyl 1-[(4-{5-[4-phenyl-5-(trifluoromethyl)-2-thienyl]-1,2,4-oxadiazol-3-yl}phenyl)methyl]-1*H*-pyrazole-4-carboxylate (D2) (85mg), ethanol (3.0ml) and 0.5M $LiOH_{(aq)}$ (1.6ml) was stirred at 40˚C. The reaction was monitored by LCMS. After 3.5 hours the temperature was raised to 45˚C and stirring continued. After a total of 7 hours the reaction mixture was allowed to cool to room temperature. Water (6ml) and 2M $HCl_{(aq)}$ (0.45ml) were then added, followed by dichloromethane (6ml). The mixture was stirred vigorously and then the dichloromethane layer was separated and evaporated to dryness. This produced a white solid, 120mg. The white solid was insoluble in 1:1 methanol and dimethylsulfoxide. The white solid was suspended in 1:1 methanol and water, sonicated and filtered, producing a white solid (purity unchanged). The solution and the solid were combined, evaporated to dryness and dissolved in methanol (100ml). The resulting solution was passed onto a PE-AX cartridge. The cartridge was washed with methanol and then 10% acetic acid in methanol. The appropriate fractions were combined and evaporated to dryness, producing 59mg of white solid. The white solid was passed onto a SAX cartridge, which was then eluted with methanol and then 10% acetic acid in methanol. The appropriate fraction was evaporated to dryness, producing 4mg of white solid. The SAX failed to catch a significant amount of desired product. The waste from the SAX was evaporated to dryness, producing 59mg of white solid. The white solid (59mg) was dissolved in methanol and passed onto a PE-AX cartridge, which was eluted with methanol and then 10% acetic acid in methanol. The appropriate fraction was evaporated to dryness, co-evaporating with ethyl acetate, producing a white solid (50mg).
MS: m/z $(M+H)^+$ 525, $C_{26}H_{19}F_3N_4O_3S$ requires 524. Impurity present in $^1H$-NMR.
The white solid was triturated with diethyl ether and dried producing **E1** as a white solid (50mg, 62%).
$^1H$-NMR (MeOD, 400MHz): δ 5.47 (2H, s), 7.44 (2H, d), 7.49 (5H, m), 7.91 (1H, s), 8.04 (1H, s), 8.14 (2H, d), 8.26 (1H, s).

**Example 2**

**Sodium 1-[(4-{5-[4-phenyl-5-(trifluoromethyl)-2-thienyl]-1,2,4-oxadiazol-3-yl}phenyl)methyl]-1*H*-pyrazole-4-carboxylate (E2)**

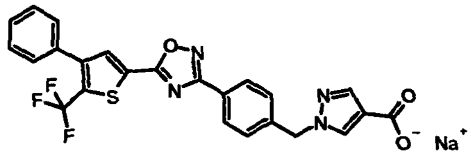

A 30mg sample of 1-[(4-{5-[4-phenyl-5-(trifluoromethyl)-2-thienyl]-1,2,4-oxadiazol-3-yl}phenyl)methyl]-1*H*-pyrazole-4-carboxylic acid (E1) was treated with an excess of 2M $NaOH_{(aq)}$, diluted with water (1ml), extracted with ethyl acetate (3x5ml), using a small volume of brine during the third extraction to aid phase separation. The combined organics were evaporated to give sodium 1-[(4-{5-[4-phenyl-5-(trifluoromethyl)-2-thienyl]-1,2,4-oxadiazol-3-yl}phenyl)methyl]-1*H*-pyrazole-4-carboxylate as a white solid (11mg, 35%).
$^1H$-NMR (MeOD, 400MHz): δ 5.46 (2H, s), 7.42 (2H, d), 7.48 (5H, m), 7.89 (1H, s), 8.03 (1H, s), 8.11 (2H, d), 8.18 (1H, s). MS: m/z $(M+H)^+$ 497, $C_{24}H_{14}F_3N_4O_3S$ requires 496.

**Example 3**

**1-[(4-{5-[4-Phenyl-5-(trifluoromethyl)-2-thienyl]-1,2,4-oxadiazol-3-yl}phenyl)methyl]-1*H*-pyrazole-5-carboxylic acid (E3)**

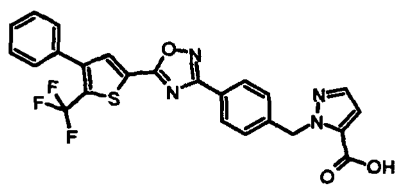

A mixture of ethyl 1-[(4-{5-[4-phenyl-5-(trifluoromethyl)-2-thienyl]-1,2,4-oxadiazol-3-yl}phenyl)methyl]-1*H*-pyrazole-5-carboxylate (D3a) (30mg), ethanol (1.1ml) and 0.5M $LiOH_{(aq)}$ (0.57ml) was stirred at 40˚C. The reaction was monitored by LCMS. After 16 hours the reaction mixture was allowed to cool to room temperature. Water (2.1ml) and 2M $HCl_{(aq)}$ (0.16ml) were then added, followed by dichloromethane (6.0ml). The mixture was stirred vigorously. The dichloromethane layer was separated and evaporated to dryness, producing 30mg of white solid. The white solid was dissolved in -7:1 methanol and dichloromethane and passed onto a SPE-AX cartridge. The cartridge was eluted with methanol and 10% acetic acid in methanol. The appropriate fraction was evaporated to dryness. No significant product eluted. Flushing the cartridge with 1:1 methanol and dichloromethane, followed by evaporation of the solvent produced a white solid (15mg).
MS: m/z $(M+H)^+$ 497, $C_{24}H_{14}F_3N_4O_3S$ requires 496. Impurity present in $^1H$-NMR.
The white solid was triturated with diethyl ether and dried producing an off white solid. The off white solid was dissolved in dichloromethane and washed with 1 M $HCl_{(aq)}$, and evaporated to dryness, producing **E3** as a white solid (8mg, 28%). $^1H$-NMR (MeOD, 400MHz): δ 5.88 (2H, s), 6.95 (1H, s), 7.34 (2H, d), 7.49 (5H, m), 7.62 (1H, s), 8.04 (1H, s), 8.07 (2H, d).

**Example 4**

**1-[(4-{5-[4-Phenyl-5-(trifluoromethyl)-2-thienyl]-1,2,4-oxadiazol-3-yl}phenyl)methyl]-1*H*-pyrazole-3-carboxylic acid (E4)**

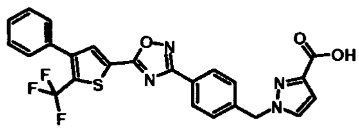

A mixture of ethyl 1-[(4-{5-[4-phenyl-5-(trifluoromethyl)-2-thienyl]-1,2,4-oxadiazol-3-yl}phenyl)methyl]-1H-pyrazole-3-carboxylate (D3b) (85mg), ethanol (3.0ml) and 0.5M $LiOH_{(aq)}$ (1.6ml) was stirred at 40˚C. The reaction was monitored by LCMS. After 16 hours the reaction mixture was allowed to cool to room temperature. Water (3.4ml) and 2M $HCl_{(aq)}$ (0.26ml) were then added, followed by dichloromethane (6.0ml). The mixture was stirred vigorously. The dichloromethane layer was separated and evaporated to dryness, producing 45mg of white solid, which was identified as E4 (99%).
$^1$H-NMR ($CDCl_3$, 400MHz): δ 5.50 (2H, s), 6.94 (1H, s), 7.39 (2H, d), 7.47 (6H, m), 7.91 (1H, s), 8.15 (2H, d). MS: m/z $(M+H)^+$ 497, $C_{24}H_{14}F_3N_4O_3S$ requires 496.

**Example 5**

**Sodium 1-[(4-{5-[4-(4-fluorophenyl)-5-(trifluoromethyl)-2-thienyl]-1,2,4-oxadiazol-3-yl}phenyl)methyl]-1*H*-pyrazole-3-carboxylate (E5)**

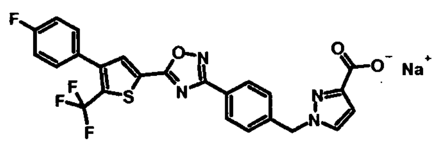

Ethyl 1-[(4-{5-[4-bromo-5-(trifluoromethyl)-2-thienyl]-1,2,4-oxadiazol-3-yl}phenyl) methyl]-1*H*-pyrazole-3-carboxylate (D8) (120mg, 0.228mmol, 1 equivalent), 4-fluorophenyl boronic acid (32mg, 0.228mmol, 1 equivalent), (tetrakistriphenylphosphine) phosphine palladium (13mg, 0.011mmol, 0.05 equivalents), 0.5M sodium carbonate aqueous solution (2.5ml) and acetonitrile (2.5ml) were irradiated at 130˚C for 20 minutes. The solvent was removed *in vacuo* and the residue taken up in water and acidified to pH1 with 5N HCl (270μl). This mixture was extracted with 3 times ethyl acetate (the biphasic mixture required filtering in order to achieve separation). The combined organics were washed with brine, dried over sodium sulphate, filtered and concentrated *in vacuo* to yield a brown solid (117mg). This was purified by column chromatography on silica (40g, 48ml) eluting in 80% ethyl acetate/*n*-hexane plus 1% acetic acid to give an off-white solid (66mg). This was suspended in methanol (1m!) and 2N NaOH (1ml) added. A white precipitate had formed. Water (2ml) was added and the suspension filtered. The precipitate was collected and dried to give sodium 1-[(4-{5-[4-(4-fluorophenyl)-5-(trifluoromethyl)-2-thienyl]-1,2,4-oxadiazol-3-yl}phenyl)methyl]-1*H*-pyrazole-3-carboxylate as an off-white solid (30mg).
$^1$H-NMR (MeOD, 400MHz): δ 5.46 (2H, s), 6.69 (1H, d), 7.24 (2H, t), 7.43 (2H, d), 7.53-7.58 (2H, m), 7.63 (1H, d), 8.05 (1H, d), 8.09 (2H, d). MS: m/z $(M+H)^+$ 515, $C_{24}H_{14}F_4N_4O_3S$ requires 515.

**Example 6**

**Sodium 1-{[4-(5-{5-chloro-6-[(1-methylethyl)oxy]-3-pyridinyl}-1,2,4-oxadiazol-3-yl)phenyl]methyl}-1*H*-pyrazole-3-carboxylate (E6)**

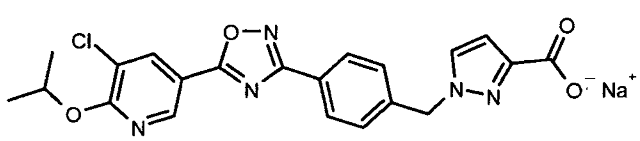

5-chloro-6-[(1-methylethyl)oxy]-3-pyridinecarboxylic acid (D10) (75 mg), EDCI (73 mg) and HOBT (52 mg) were dissolved

in DMF (3.5 ml) and stirred at RT for 10 minutes. Ethyl 1-({4-[(hydroxyamino)(imino)methyl]phenyl}methyl)-1*H*-pyrazole-3-carboxylate (D7) (100 mg) was added and stirring continued at RT for 1 hour then at 80 ˚C overnight. The reaction mixture was then evaporated to dryness, treated with EtOH (10 ml) and 2 M aqueous NaOH (10 ml) and stirred at 50 ˚C for 2 hours. The reaction mixture was evaporated to remove the EtOH and the precipitate removed by filtration then washed with $H_2O$ to give the title compound (10 mg) as an off white solid. δH (MeOD, 400MHz): 1.42 (6H, d), 5.45 (2H, s), 5.51 (1H, quintet), 6.69 (1H, d), 7.41 (2H, d), 7.63 (1H, d), 8.09 (2H, d), 8.46 (1H, d), 8.88 (1H, d). MS (ES): $C_{21}H_{18}ClN_5O_4$ requires 439.86; found 440 (M+H).

Example 7

**Sodium 1-[(4-{5-[4-(methyloxy)-3-(trifluoromethyl)phenyl]-1,2,4-oxadiazol-3-yl}phenyl)methyl]-1*H*-pyrazole-3-carboxylate (E7)**

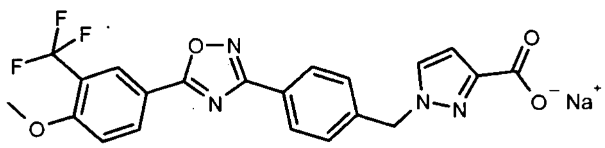

4-(methyloxy)-3-(trifluoromethyl)benzoic acid (76 mg), EDCI (73 mg) and HOBT (52 mg) were dissolved in DMF (3.5 ml) and stirred at RT for 10 minutes. Ethyl 1-({4-[(hydroxyamino)(imino)methyl]phenyl}methyl)-1*H*-pyrazole-3-carboxylate (D7) (100 mg) was added and stirring continued at RT for 1 hour then at 80 ˚C overnight. The reaction mixture was then evaporated to dryness, re-dissolved in $H_2O$, extracted with EtOAc (x 2) and the combined EtOAc solutions evaporated to dryness. The residue was treated with EtOH (10 ml) and 2 M aqueous NaOH (10 ml) then stirred at 50 ˚C for 2 hours. The reaction mixture was then evaporated to remove the EtOH and the precipitate removed by filtration then washed with $H_2O$ to give the title compound CQ107723-114A1 (47 mg) as an off white solid. δH (MeOD, 400MHz): 4.04 (3H, s), 5.46 (2H, s), 6.69 (1H, d), 7.40-7.46 (3H, m), 7.64 (1H, d), 8.10 (2H, d), 8.38 (1H, s), 8.42 (1H, dd). MS (ES): $C_{21}H_{15}F_3N_4O_4$ requires 444.37; found 445 (M+H).

**Example 8**

**Sodium 1-{[4-(5-{3-chloro-4-[(1-methylethyl)oxy]phenyl}-1,2,4-oxadiazol-3-yl)phenyl]methyl}-1*H*-pyrazole-3-carboxylate (E8)**

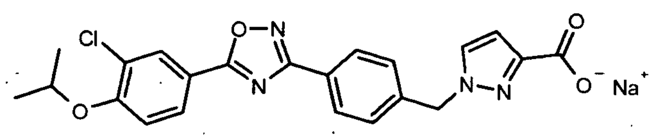

Ethyl 1-{[4-(5-{3-chloro-4-[(1-methylethyl)oxy]phenyl}-1,2,4-oxadiazol-3-yl)phenyl] methyl}-1*H*-pyrazole-3-carboxylate (D14) was treated with EtOH (10 ml) and 2 M aqueous NaOH (10 ml) and stirred overnight at 50 ˚C. The reaction mixture was then evaporated to dryness, acidified to pH = 2 with 2 M aqueous HCl and extracted with EtOAc (x 3). The combined organic solutions were evaporated to dryness and the residue purified by MDAP to give the title compound (23 mg) as a white solid. δH (MeOD, 400MHz): 1.44 (6H, s), 4.72 (1H, quintet), 5.47 (2H, s), 6.93 (1H, d), 7.06 (1H, d), 7.37 (2H, d), 7.47 (1H, d), 8.05 (1H, dd), 8.16 (2H, d), 8.23 (1H, d). MS (ES): $C_{22}H_{19}ClN_4O_4$ requires 438.87; found 439 (M+H).

**Example 9**

**Sodium 1-{[4-(5-{3-chloro-4-[(trifluoromethyl)oxy]phenyl}-1,2,4-oxadiazol-3-yl)phenyl]methyl}-1*H*-pyrazole-3-carboxylate (E9)**

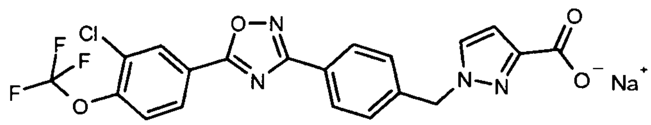

3-chloro-4-[(trifluoromethyl)oxy]benzoic acid (83 mg) was used in a similar procedure to that described above for E7, to give the title compound (75 mg) as a white solid. δH (MeOD, 400MHz): 5.46 (2H, s), 6.69 (1H, d), 7.43 (2H, d), 7.63 (1H, d), 7.71 (1H, dq), 8.11 (2H, d), 8.27 (1H, dd), 8.43 (1H, d). MS (ES): $C_{20}H_{12}ClF_3N_4O_4$ requires 464.79; found 465 (M+H).

**Example 10**

**Sodium 1-{[4-(5-{3-cyano-4-[(1-methylethyl)oxy]phenyl}-1,2,4-oxadiazol-3-yl)phenyl]methyl}-1*H*-pyrazole-3-carboxylate (E10)**

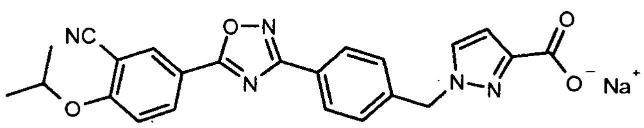

3-Cyano-4-[(1-methylethyl)oxy]benzoic acid (can be prepared as described in WO2005/58848) (124 mg) was used in a similar procedure to that described above for E7 to give the title compound (100 mg) as a white solid. δH (MeOD, 400MHz): 1.45 (6H, d), 4.94 (1H, quintet), 5.45 (2H, s), 6.69 (1H, d), 7.40-7.45 (3H, m), 7.63 (1H, d), 8.09 (2H, d), 8.41 (1H, dd), 8.44 (1H, d). MS (ES): $C_{23}H_{19}N_5O_4$ requires 429.43; found 430 (M+H).

**Examples 11 and 12**

**1-[(4-{5-[2'-fluoro-2-(trifluoromethyl)-4-biphenylyl]-1,2,4-oxadiazol-3-yl}phenyl)methyl]-1H-pyrazole-3-carboxylic acid sodium salt (E11) and 1-[(4-{5-[2'-fluoro-2-(trifluoromethyl)-4-biphenylyl]-1,2,4-oxadiazol-3-yl}phenyl)methyl]-1H-pyrazole-3-carboxylic acid (E12)**

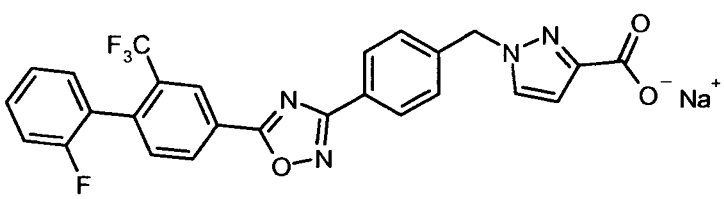

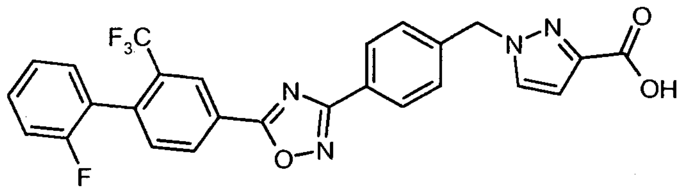

Methyl 1-[(4-{5-[2'-fluoro-2-(trifluoromethyl)-4-biphenylyl]-1,2,4-oxadiazol-3-yl}phenyl) methyl]-1H-pyrazole-3-carboxylate (D28) (73 mg, 0.140 mmol) was dissolved in ethanol (10 ml) at 40 °C and aqueous sodium hydroxide (2M, 1 ml, 2.000 mmol) was added. The reaction was heated at 40 °C for 3 hours and then left to stand at room temperature for 18 h. The mixture was concentrated *in vacuo* to give a white suspension in water. The solid was filtered off, but redissolved when washing with a small amount of water. The filtrate was concentrated and the mixture refiltered, washing with a very small amount of water. The resulting gel was triturated with ethanol, filtered and dried in a vacuum oven to give 1-[(4-{5-[2'-fluoro-2-(trifluoromethyl)-4-biphenylyl]-1,2,4-oxadiazol-3-yl}phenyl)methyl]-1H-pyrazole-3-carboxylic acid sodium salt (5 mg, 9.41 μmol) as a white solid. MS ($ES^+$): $C_{26}H_{16}F_4N4O_3$ requires 508; found 509 ($M+H^+$).

The filtrates from this experiment were combined and concentrated *in vacuo* and separated between 2M HCl (aqueous, 20 mL) and dichloromethane (30 mL). The aqueous was extracted with further dichloromethane (30 mL) before the combined organic phase was dried (phase separator) and concentrated *in vacuo* and dried in a vacuum oven to give 1-[(4-{5-[2'-fluoro-2-(trifluoromethyl)-4-biphenylyl]-1,2,4-oxadiazol-3-yl}phenyl)methyl]-1H-pyrazole-3-carboxylic acid (15 mg, 0.030 mmol) as a white solid. δH ($CDCl_3$, 400 MHz): 8.63 (1H, d), 8.41 (1H, dd), 8.20 (2H, d), 7.57 (1H, d), 7.48 (1H, d), 7.45 (1H, m), 7.41 (2H, d), 7.29 (1H, t), 7.24 (1H, qd), 7.18 (1H, t), 6.95 (1H, d), 5.51 (2H, s) ppm. MS ($ES^+$): $C_{26}H_{16}F_4N4O_3$ requires 508; found 509 ($M+H^+$).

**<u>Example 13</u> 1-[(4-{5-[4-cyclohexyl-3-(trifluoromethyl)phenyl]-1,2,4-oxadiazol-3-yl}phenyl)methyl]-1H-pyrazole-3-carboxylic acid sodium salt (E13)**

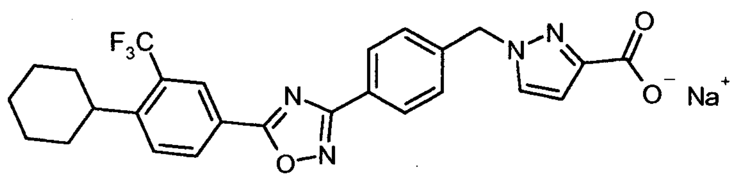

To a solution of methyl 1-[(4-{5-[4-cyclohexyl-3-(trifluoromethyl)phenyl]-1,2,4-oxadiazol-3-yl}phenyl)methyl]-1H-pyrazole-3-carboxylate (D29) (162 mg, 0.317 mmol) in ethanol (15 ml) at 40 °C was added sodium hydroxide (2M, 1 ml, 2.000 mmol) and the solution heated at 40 °C for 18 h. The reaction mixture was concentrated to about 5 mL *in vacuo* and the resultant solid filtered off, washed with water and dried in a vacuum oven over a weekend to give the title compound as a white solid (142 mg, 0.273 mmol). δH (MeOD, 400 MHz): 8.43 (1H, d), 8.39 (1H, dd), 8.12 (2H, d), 7.84 (1H, d), 7.64 (1H, d), 7.43 (2H, d), 6.70 (1H, d), 5.46 (2H, s), 3.02 (1H, t), 1.91 (2H, m), 1.87-1.78 (3H, m), 1.62 (2H, q), 1.51-1.37 (3H, m) ppm. MS ($ES^+$): $C_{26}H_{23}F_3N_4O_3$ requires 496; found 497 ($M+H^+$).

**<u>Example 14</u>**

**1-[(4-{5-[4-(1-cyclohexen-1-yl)-3-(trifluoromethyl)phenyl]-1,2,4-oxadiazol-3-yl}phenyl)methyl]-1H-pyrazole-3-carboxylic acid sodium salt (E14)**

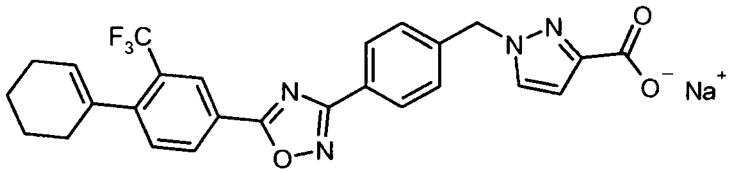

Methyl 1-[(4-{5-[4-(1-cyclohexen-1-yl)-3-(trifluoromethyl)phenyl]-1,2,4-oxadiazol-3-yl} phenyl)methyl]-1H-pyrazole-3-carboxylate (D30) (67 mg, 0.132 mmol) was dissolved in ethanol (5 ml) at 40 ˚C and sodium hydroxide (2M, 0.5 ml, 1.000 mmol) was added. The reaction was heated at 40 ˚C for 18 h. The reaction mixture was concentrated *in vacuo* and the white solid filtered off and washed with water. On filtration, much compound redissolved and passed through, so solid and filtrate were combined and separated between dichloromethane (10 mL) and 2M HCl (3 mL). The aqueous phase was extracted with further dichloromethane (10 mL). The organic phases were isolated by phase separator, combined and the solvent removed *in vacuo.* The solid was then dissolved in acetonitrile and water with addition of an equimolar amount of sodium hydroxide before the solution was freeze-dried to give the title compound as a white solid (26 mg, 0.050 mmol). δH (MeOD, 400 MHz): 8.46 (1H, d), 8.36 (1H, dd), 8.12 (2H, d), 7.64 (1H, d), 7.54 (1H, d), 7.43 (2H, d), 6.70 (1H, d), 5.66 (1H, brs), 5.46 (2H, s), 2.31-2.25 (2H, m), 2.23-2.17 (2H, m), 1.84-1.77 (2H, m), 1.76-1.69 (2H, m) ppm. MS ($ES^+$): $C_{26}H_{21}F_3N_4O_3$ requires 494; found 495 ($M+H^+$).

<u>**Example 15**</u>

**1-[(4-{5-[4-(2-methylpropyl)-3-(trifluoromethyl)phenyl]-1,2,4-oxadiazol-3-yl}phenyl)methyl]-1H-pyrazole-3-carboxylic acid sodium salt (E15)**

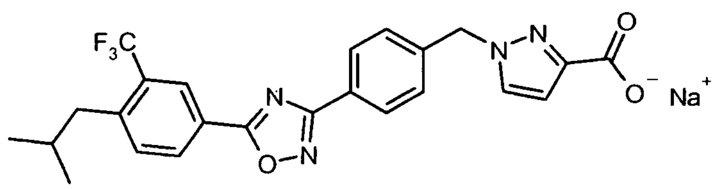

Methyl 1-[(4-{5-(4-(2-methylpropyl)-3-(trifluoromethyl)phenyl]-1,2,4-oxadiazol-3-yl} phenyl)methyl]-1H-pyrazole-3-carboxylate (D31) (38 mg, 0.078 mmol) was dissolved in ethanol (8 ml) and sodium hydroxide (2M, 0.5 ml, 1.000 mmol) was added. The reaction was heated at 40 ˚C for 18 h. The reaction mixture was concentrated *in vacuo* and the white solid filtered off and washed with water. On filtration, much compound redissolved and passed through, so solid and filtrate were combined and separated between dichloromethane (10 mL) and 2M HCl (3 mL). The aqueous phase was extracted with further dichloromethane (10 mL). The organic phases were isolated by phase separator, combined and the solvent removed *in vacuo.* The solid was then dissolved in acetonitrile and water with addition of an equimolar amount of sodiium hydroxide before the solution was freeze-dried to give the title compound as a pale brown solid (18 mg, 0.036 mmol). δH (MeOD, 400 MHz): 8.46 (1H, d), 8.37 (1H, dd), 8.12 (2H, d), 7.71 (1H, d), 7.63 (1H, d), 7.45 (2H, d), 6.70 (1H, d), 5.46 (2H, s), 2.79 (2H, d), 2.06 (1H, m), 0.98 (6H, d) ppm. MS ($ES^+$): $C_{24}H_{21}F_3N_4O_3$ requires 470; found 471 ($M+H^+$).

<u>**Example 16**</u>

**Sodium 1-({4-[5-(2-ethyl-4-biphenylyl)-1,2,4-oxadiazol-3-yl]phenyl}methyl)-1*H*-pyrazole-3-carboxylate (E16)**

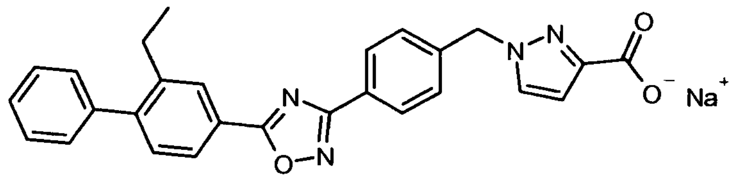

To a suspension of methyl 1-({4-[5-(2-ethyl-4-biphenylyl)-1,2,4-oxadiazol-3-yl]phenyl} methyl)-1*H*-pyrazole-3-carboxylate (D32) (131 mg, 0.28 mmol) in ethanol (12.7 mL) was added sodium hydroxide (2M, 1.27 mL, 2.8 mmol) and the mixture heated to 55 ˚C to dissolve and then the reaction heated at 40 ˚C for 1 h. The solvent was partially evaporated and the resultant precipitate filtered off, washed with water and dried to give the title compound as a white solid (9 mg, 0.02 mmol). δH (DMSO, 400 MHz): 8.14 (1H, s), 8.10-8.04 (3H, m), 7.69 (1H, d), 7.53-7.48 (2H, m), 7.47-7.42 (4H, m), 7.39 (2H, d), 6.37 (1H, d), 5.41 (2H, s), 2.69 (2H, q), 1.10 (3H, t) ppm. MS ($ES^+$): $C_{27}H_{22}N_4O_3$ requires 450; found 451 ($M+H^+$).

**Example 17**

**Sodium 1-[(4-{5-[4-[(1-methylethyl)oxy]-3-(trifluoromethyl)phenyl]-1,2,4-oxadiazol-3-yl}phenyl)methyl]-1*H*-pyrazole-3-carboxylate (E17)**

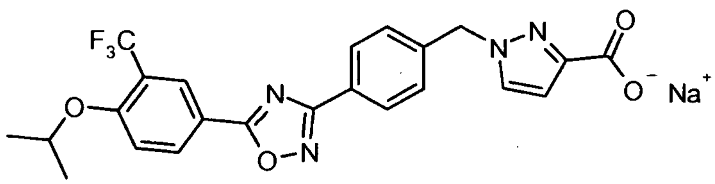

To a suspension of methyl 1-[(4-{5-[4-[(1-methylethyl)oxy]-3-(trifluoromethyl)phenyl]-1,2,4-oxadiazol-3-yl}phenyl)methyl]-1*H*-pyrazole-3-carboxylate (E33) (124 mg, 0.26 mmol) in ethanol (12 mL) was added sodium hydroxide (2M, 1.2 mL, 2.4 mmol) and the mixture heated to 50 ˚C to dissolve. The reaction was then heated at 40 ˚C for 1 h. The solvent was evaporated and the residue partitioned between ethyl acetate (25 mL) and water (25 mL) and the latter acidified with 2M HCl. The aqueous layer was extracted with further ethyl acetate (2 x 25 mL) and the combined organic extracts dried (phase separator) and evaporated. The resultant solid was dissolved in acetonitrile and water with an equimolar amount of 2M sodium hydroxide and the solution freeze dried to give the title compound as a white solid (109 mg, 0.22 mmol). δH (DMSO, 400 MHz): 8.39 (1H, dd), 8.29 (1H, d), 8.05 (2H, d), 7.68 (1H, d), 7.59 (1H, d), 7.42 (2H, d), 6.37 (1H, d), 5.41 (2H, s), 4.97 (1H, septet), 1.35 (6H, d) ppm. MS ($ES^+$): $C_{23}H_{19}F_3N_4O_4$ requires 472; found 473 ($M+H^+$).

**Example 18** **Sodium 1-({4-[5-(2-ethyl-2'-fluoro-4-biphenylyl)-1,2,4-oxadiazol-3-yl]phenyl}methyl)-1H-pyrazole-3-carboxylate (E18)**

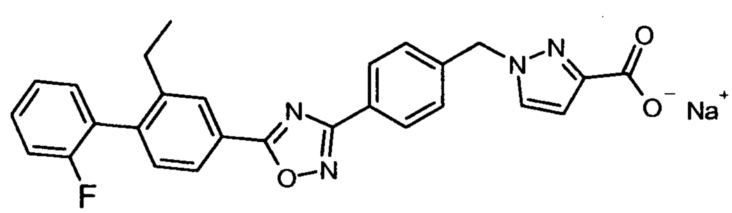

To a suspension of methyl 1-({4-[5-(2-ethyl-2'-fluoro-4-biphenylyl)-1,2,4-oxadiazol-3-yl]phenyl}methy)-1*H*-pyrazole-3-carboxylate (E34) (48 mg, 0.10 mmol) in ethanol (4.6 mL) was added sodium hydroxide (2M, 0.46 mL, 0.92 mmol)

and the mixture heated to 50 ˚C to dissolve. The reaction was then heated at 40 ˚C for 1 h before it was concentrated and the residue partitioned between ethyl acetate (10 mL) and water (10 mL) with the latter acidified with 2M HCl. The aqueous layer was then extracted with further ethyl acetate (2 x 10 mL) before the combined organic extracts were dried (phase separator) and evaporated. The residue was dissolved in acetonitrile and water with an equimolar amount of 2M sodium hydroxide and the solution freeze dried to give the title compound (44 mg, 0.09 mmol). δH (DMSO, 400 MHz): 8.17 (1H, d), 8.10-8.07 (3H, m), 7.69(1H, d), 7.52 (1H, m), 7.47 (1H, d), 7.44 (2H, d), 7.42-7.32 (3H, m), 6.37 (1H, d), 5.42 (2H, s), 2.58 (2H, q), 1.07 (3H, t) ppm. MS (ES$^+$): $C_{27}H_{21}FN_4O_3$ requires 468; found 469 (M+H$^+$).

**Example 19**

**Sodium 1-[(4-{5-[2-(trifluoromethyl)-4-biphenylyl]-1,2,4-oxadiazol-3-yl}phenyl) methyl]-1*H*-pyrazole-3-carboxylate (E19)**

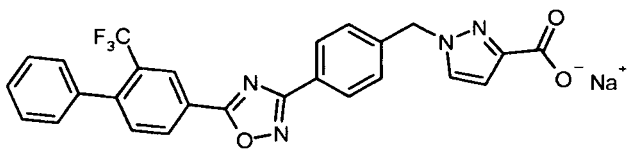

To a suspension of methyl 1-[(4-{5-[2-(trifluoromethyl)-4-biphenylyl]-1,2,4-oxadiazol-3-yl}phenyl)methyl]-1*H*-pyrazole-3-carboxylate (D35) (164 mg, 0.33 mmol) in ethanol (20 mL) and the mixture heated to 60 ˚C to dissolve. Sodium hydroxide (2M, 2.0 mL, 4.0 mol) was added and the reaction heated to at 40 ˚C for 1 h. The reaction was left to stand at room temperature for 18 h before the mixture was partially concentrated and the resultant precipitate filtered off and washed with water to give the title compound as a white solid (105 mg, 0.21 mmol). δH (DMSO, 400 MHz): 8.52-8.48 (2H, m), 8.10 (2H, d), 7.75-7.71 (2H, m), 7.54-7.50 (3H, m), 7.46 (2H, d), 7.42-7.39 (2H, m), 6.43 (1H, d), 5.43 (2H, s) ppm. MS (ES$^+$): $C_{26}H_{17}F_3N4O_3$ requires 490; found 491 (M+H$^+$).

**Example 20**

**1-[(3-chloro-4-{5-[4-phenyl-5-(trifluoromethyl)-2-thienyl]-1,2,4-oxadiazol-3-yl}phenyl)methyl]-1H-pyrazole-3-carboxylic acid sodium salt (E20)**

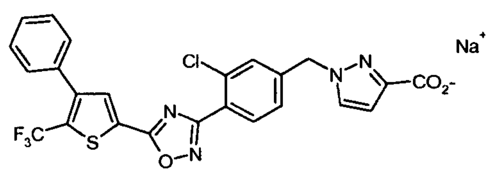

Ethyl 1-[(3-chloro-4-{5-[4-phenyl-5-(trifluoromethyl)-2-thienyl]-1,2,4-oxadiazol-3-yl} phenyl)methyl]-1H-pyrazole-3-carboxylate (D38) (54mg, 0.01 mmol), in methanol (40ml) and sodiumhydroxide solution 2M (4ml) was warmed at 30˚C overnight. The reaction mixture was then heated at 60˚C for 5 hours, the solvent was removed in vacuo and water added (~10ml), the mixture was scratched down and left to stand for 2 hours, the resultant suspension was filtered and the solid dried overnight under vacuum at 60˚C to yield the title compound as a yellow solid (33mg, 0.006 mmol). δH (DMSO, 400 MHz): 8.26 (1H, s), 7.98, (1H, d), 7.72, (1H, s), 7.49-7.60 (6H, m), 7.39-7.47, (2H, m), 6.36, (1H, s), 5.42, (2H, s). MS (ES$^+$): requires $C_{24}H_{14}{}^{35}ClF_3N_4O_3S$ 530; found 531 (M+H$^+$).

**Example 21**

**1-[(2-chloro-4-{5-[4-phenyl-5-(trifluoromethyl)-2-thienyl]-1,2,4-oxadiazol-3-yl}phenyl)methyl]-1H-pyrazole-3-carboxylic acid sodium salt (E21)**

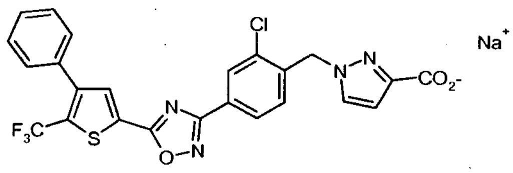

Ethyl 1-[(2-chloro-4-{5-[4-phenyl-5-(trifluoromethyl)-2-thienyl]-1,2,4-oxadiazol-3-yl} phenyl)methyl]-1H-pyrazole-3-carboxylate (D41) (85mg, 0.15 mmol), in methanol (10ml) and sodium hydroxide solution 2M (2ml) was stirred at room temperature for 4 hours. A further 40ml of methanol and 10ml of sodium hydroxide were added and the mixture heated at 40˚C over night. The solvent was removed in vacuo and water added (~15ml), the mixture was scratched down and left to stand for 2 hours, the resultant suspension was filtered and the solid dried overnight under vacuum at 50˚C. the title compound as a white solid (65mg, 0.12 mmol). δH (DMSO, 400 MHz): 8.27 (1H, s), 8.11 (1H, s), 7.99, (1H, d), 7.70, (1H, s), 7.50-7.61 (5H, m), 6.98, (1H, d), 6.39, (1H, s), 5.51, (2H, s). MS ($ES^+$): requires $C_{24}H_{14}{}^{35}ClF_3N_4O_3S$ 530; found 531 ($M+H^+$).

**Example 22**

**1-{[3-bromo-4-(5-{3-cyano-4-[(1-methylethyl)oxy]phenyl}-1,2,4-oxadiazol-3-yl)phenyl]methyl}-1H-pyrazole-3-carboxylic acid sodium salt (E22)**

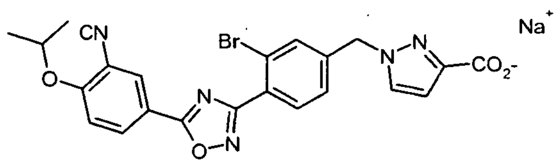

Ethyl 1-{[3-bromo-4-(5-{3-cyano-4-[(1-methylethyl)oxy]phenyl}-1,2,4-oxadiazol-3-yl) phenyl]methyl}-1H-pyrazole-3-carboxylate (D45) (15mg, 0.03 mmol), was stirred in a mixture of methanol (1.5ml) and 2M aq sodium hydroxide solution (0.5ml) at 30˚C for 3 hours. Water (~7ml) was added and the mixture scratched down, this was allowed to stand over the weekend and the solid filtered and dried under vacuum at 60˚C. To yield the title compound as a yellow solid (9mg, 0.018mmol). δH (DMSO, 400 MHz): 8.50 (1H, d), 8.39 (1H, dd), 7.89 (1H, d), 7.69 (1H, s), 7.66 (1H, s), 7.56 (1H, d), 7.40 (1H, d), 6.34 (1H, s), 5.40 (2H, s), 4.98 (1H, sept), 1.38 (6H, d). MS ($ES^+$): requires $C_{23}H_{18}{}^{79}BrN_5O_4$ 507; found 508 ($M+H^+$).

**GTPγS binding assay**

Rat Basophilic Leukaemia cells (RBL) stably expressing S1P1 receptor were grown to 80% confluency before being harvested into 10ml Phospho-Buffered Saline (PBS) and centrifuged at 1200rpm for 5 minutes. After removal of the supernatant, the pellet was re-suspended and homogenised in 20 volumes assay buffer (20mM HEPES pH 7.4, 100mM NaCl, 10mM MgCL.6H2O, 10μM GDP Saponin 10μg/ml). The membrane suspension was further centrifuged for 20 minutes at 20,000rpm re-homogenised and spun again. Following the second centrifugation the pellet was re-suspended in an appropriate volume (1ml for each flask of cells) and assayed for protein concentration.

Concentrated stock of S1 P was sonicated before serial dilutions were prepared from a starting concentration of $10^{-5}$ M. Diluted membranes (10μg/well) were incubated with various concentrations of S1P and 0.3nM $^{35}S$-GTPγS (NEN; specific activity1250 Ci/mmol) in 96 deep well plates. Binding was performed at 30˚C for 45 minutes and terminated by harvesting the membranes onto GF/B filter plates using a Packard Universal Harvester. After drying the plates for

45 minutes, 50μl of Microscint 0 was added to each well and binding measured on a Topcount NXT

(Perkin Elmer). Data was analysed using Graphpad Prism 4 and expressed as percentage stimulation above basal. EC50 values were defined as the concentration of agonist required to give 50% of the maximal stimulation.

**Membrane preparation (alternative method)**

For membrane preparations all steps were performed at 4˚C. Rat hepatoma cells stably expressing the human S1 P1 receptor or Rat Basophilic Leukaemia cells (RBL) stably expressing human S1 P3 receptor were grown to 80% confluency before being harvested into 10ml Phospho-Buffered Saline (PBS) and centrifuged at 1200rpm for 5 minutes. After removal of the supernatant, the pellet was re-suspended and cells were homogenised within a glass Waring blender for 2 bursts of 15secs in 200mls of buffer (50mM HEPES, 1 mM leupeptin, 25μg/ml bacitracin, 1 mM EDTA, 1 mM PMSF, 2μM pepstatin A). The blender was plunged into ice for 5 mins after the first burst and 10-40 mins after the final burst to allow foam to dissipate. The material was then spun at 500g for 20 mins and the supernatant spun for 36 mins at 48,000g. The pellet was resuspended in the same buffer as above but without PMSF and pepstatin A. The material was then forced through a 0.6mm needle, made up to the required volume, (usually x4 the volume of the original cell pellet), aliquoted and stored frozen at -80˚C.

**S1P1 GTPγS assay (alternative method)**

Human S1P1 rat hepatoma membranes (1.5μg/well) were adhered to a wheatgerm agglutinin (WGA)-coated scintillation proximity assay (SPA) beads (0.125mg/well) in assay buffer (HEPES 20mM, $MgCl_2$ 10mM, NaCl 100mM and pH adjusted to 7.4 using KOH 5M, GDP 10μM FAC (final assay concentration) and saponin 90μg/ml FAC was also added).

After 30 minutes pre-coupling on ice the bead and membrane suspension was dispensed into a white Greiner polypropylene LV384-well plate (5μl/well), containing 0.1μl of the compound. 5μl/well [$^{35}$S]-GTPγS (0.5nM final radioligand conc) made up in assay buffer was then added to agonist plates. The final assay cocktail (10.1 μl) was then centrifuged at 1000rpm for 5 minutes then read immediately on a Viewlux reader.

All test compounds were dissolved in DMSO at a concentration of 10mM and were prepared in 100% DMSO using a 1 in 4 dilution step to provide 11 point dose response curves. The dilutions were transferred to the assay plates ensuring that the DMSO concentration was constant across the plate for all assays.

All data was normalized to the mean of 16 high and 16 low control wells on each plate. A four parameter curve fit was then applied.

Exemplified compounds of the invention have a $pEC_{50}$ > 5 in the GTPγS binding assay.

**S1P3 GTPγS assay**

S1P3 membranes from rat basophilic leukaemia cells (RBL-2H3)(1.5μg/well) were adhered to WGA-coated SPA beads (0.125mg/well) in assay buffer (HEPES 20mM, $MgCl_2$ 3mM, NaCl 100mM and pH adjusted to 7.4 using KOH 5M), GDP 10μM FAC and saponin 90μg/ml FAC was also added).

After 30 minutes pre-coupling on ice the bead and membrane suspension was dispensed into a white Greiner polypropylene LV384-well plate (5μl/well), containing 0.1μl of the compound. 5μl/well [$^{35}$S]-GTPγS (0.5nM final radioligand conc) made up in assay buffer was then added to agonist plates. The final assay cocktail (10.1μl) was centrifuged at 1000rpm for 5 minutes then read immediately on a Viewlux reader.

All test compounds were dissolved in DMSO at a concentration of 10mM and were prepared in 100% DMSO using a 1 in 4 dilution step to provide 11 point dose response curves. The dilutions were transferred to the assay plates ensuring that the DMSO concentration was constant across the plate for all assays.
All data was normalized to the mean of 16 high and 16 low control wells on each plate. A four parameter curve fit was then applied.

Exemplified compounds tested in this assay had a pEC50 < 6, some had a pEC50 <5.

**Yeast assay**

Yeast (*Saccharomyces cerevisiae*) cells expressing the human S1P1 receptor were generated by integration of an expression cassette into the *ura3* chromosomal locus of yeast strain MMY23. This cassette consisted of DNA sequence encoding the human S1 P1 receptor flanked by the yeast GPD promoter to the 5' end of S1 P1 and a yeast transcriptional terminator sequence to the 3' end of S1P1. MMY23 expresses a yeast/mammalian chimeric G-protein alpha subunit in which the C-terminal 5 amino acids of Gpa1 are replaced with the C-terminal 5 amino acids of human Gαi1/2 (as described in Brown *et al.* (2000), *Yeast* **16**:11-22). Cells were grown at 30˚C in liquid Synthetic Complete

(SC) yeast media (Guthrie and Fink (1991), Methods in Enzymology, Vol. 194) lacking uracil, tryptophan, adenine and leucine to late logarithmic phase (approximately 6 $OD_{600}$/ml).

Agonists were prepared as 10 mM solutions in DMSO. $EC_{50}$ values (the concentration required to produce 50% maximal response) were estimated using 4 fold dilutions (BiomekFX, Beckman) into DMSO. Agonist solutions in DMSO (1% final assay volume) were transferred into black microtitre plates from Greiner (384-well). Cells were suspended at a density of 0.2 $OD_{600}$/ml in SC media lacking histidine, uracil, tryptophan, adenine and leucine and supplemented with 0.1mM 3-aminotriazole, 0.1M sodium phosphate pH 7.0, and 10μM fluorescein di-p-D-glucopyranoside (FDGlu). This mixture (50ul per well) was added to agonist in the assay plates (Multidrop 384, Labsystems). After incubation at 30°C for 24 hours, fluorescence resulting from degradation of FDGlu to fluorescein due to exoglucanase, an endogenous yeast enzyme produced during agonist-stimulated cell growth, was determined using a fluorescence microtitre plate reader (Tecan Spectrofluor or LJL Analyst excitation wavelength: 485nm; emission wavelength: 535nm). Fluorescence was plotted against compound concentration and iteratively curve fitted using a four parameter fit to generate a concentration effect value. Efficacy ($E_{max}$) was calculated from the equation

$$E_{max} = Max_{[compound\ X]} - Min_{[compound\ X]} / Max_{[S1P]} - Min_{[S1P]} \times 100\%$$

where $Max_{[compound\ X]}$ and $Min_{[compound\ X]}$ are the fitted maximum and minimum respectively from the concentration effect curve for compound X, and $Max_{[S1P]}$ and $Min_{[S1P]}$ are the fitted maximum and minimum respectively from the concentration effect curve for Sphingosine-1-Phosphate (available from Sigma). Equieffective molar ratio (EMR) values were calculated from the equation

$$EMR = EC_{50\ [compound\ X]} / EC_{50\ [S1P]}$$

Where $EC_{50\ [compound\ X]}$ is the $EC_{50}$ of compound X and $EC_{50\ [S1P]}$ is the $EC_{50}$ of S1P.

Exemplified compounds tested in this assay have a $pEC_{50}$ > 5.

## Claims

**1.** A compound of formula (I) or a pharmaceutically acceptable salt thereof:

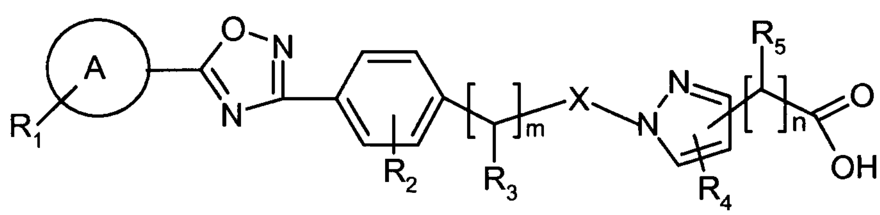

(I)

wherein:

A is a phenyl or a 5 or 6-membered heteroaryl ring;
$R_1$ is hydrogen or up to three substituents selected from halogen, $C_{(1-4)}$alkyl, $C_{(5-6)}$cycloalkyl, $C_{(3-6)}$cycloalkenyl, $C_{(1-4)}$alkoxy, trifluoromethoxy, difluoromethoxy, trifluoromethyl, cyano,
optionally substituted phenyl or optionally substituted 5 or 6 membered heteroaryl rings;
$R_2$ is hydrogen or up to three substituents selected from halogen, $C_{(1-4)}$alkyl, $C_{(1-4)}$alkoxy, trifluoromethoxy, difluoromethoxy, trifluoromethyl or cyano;
$R_3$, $R_4$ and $R_5$ are each independently selected from hydrogen, fluoro and methyl;
X is an optionally substituted $CH_2$ or a direct bond;

m is 0 to 1; and
n is 0 to 2.
wherein, when $R_1$ comprises a substituted phenyl or 5 or 6 membered heteroaryl ring, it is substituted by up to three substituents independently selected from halogen, $C_{(1-4)}$alkyl, $C_{(1-4)}$alkoxy, trifluoromethoxy, difluoromethoxy, trifluoromethyl and cyano.

**2.** A compound of formula (I) or a pharmaceutically acceptable salt thereof, wherein:

A is optionally substituted pyridyl, phenyl or thiophene; and/or
$R_1$ is optionally substituted phenyl, trifluoromethyl, trifluoromethoxy, chloro, isopropoxy, isobutyl, methoxy, cyano, cyclohexyl, cyclohexenyl or ethyl.; and/or
$R_2$ is hydrogen, chloro or bromo; and/or
$R_3$, $R_4$ and $R_5$ are each hydrogen; and/or
X is $CH_2$; and/or
m is 0; and/or
n is 0.

**3.** A compound selected from:

1-[(4-{5-[4-Phenyl-5-(trifluoromethyl)-2-thienyl]-1,2,4-oxadiazol-3-yl}phenyl)methyl]-1*H*-pyrazole-4-carboxylic acid
1-[(4-{5-[4-Phenyl-5-(trifluoromethyl)-2-thienyl]-1,2,4-oxadiazol-3-yl}phenyl)methyl]-1*H*-pyrazole-5-carboxylic acid
1-[(4-{5-[4-Phenyl-5-(trifluoromethyl)-2-thienyl]-1,2,4-oxadiazol-3-yl}phenyl)methyl]-1*H*-pyrazole-3-carboxylic acid
1-[(4-{5-[4-(4-fluorophenyl)-5-(trifluoromethyl)-2-thienyl]-1,2,4-oxadiazol-3-yl}phenyl)methyl]-1*H*-pyrazole-3-carboxylic acid
1-{[4-(5-{5-chloro-6-[(1-methylethyl)oxy]-3-pyridinyl}-1,2,4-oxadiazol-3-yl)phenyl]methyl}-1*H*-pyrazole-3-carboxylic acid
1-[(4-{5-[4-(methyloxy)-3-(trifluoromethyl)phenyl]-1,2,4-oxadiazol-3-yl}phenyl)methyl]-1*H*-pyrazole-3-carboxylic acid
1-{[4-(5-{3-chloro-4-[(1-methylethyl)oxy]phenyl}-1,2,4-oxadiazol-3-yl)phenyl]methyl}-1*H*-pyrazole-3-carboxylic acid
1-{[4-(5-{3-chloro-4-[(trifluoromethyl)oxy]phenyl}-1,2,4-oxadiazol-3-yl)phenyl]methyl}-1*H*-pyrazole-3-carboxylic acid
1-{[4-(5-{3-cyano-4-[(1-methylethyl)oxy]phenyl}-1,2,4-oxadiazol-3-yl)phenyl]methyl}-1*H*-pyrazole-3-carboxylic acid
1-[(4-{5-[2'-fluoro-2-(trifluoromethyl)-4-biphenylyl]-1,2,4-oxadiazol-3-yl}phenyl)methyl]-1H-pyrazole-3-carboxylic acid
1-[(4-{5-[2'-fluoro-2-(trifluoromethyl)-4-biphenylyl]-1,2,4-oxadiazol-3-yl}phenyl)methyl]-1H-pyrazole-3-carboxylic acid
1-[(4-{5-[4-cyclohexyl-3-(trifluoromethyl)phenyl]-1,2,4-oxadiazol-3-yl}phenyl)methyl]-1H-pyrazole-3-carboxylic acid
1-[(4-{5-[4-(1-cyclohexen-1-yl)-3-(trifluoromethyl)phenyl]-1,2,4-oxadiazol-3-yl}phenyl)methyl]-1H-pyrazole-3-carboxylic acid
1-[(4-{5-[4-(2-methylpropyl)-3-(trifluoromethyl)phenyl]-1,2,4-oxadiazol-3-yl}phenyl)methyl]-1H-pyrazole-3-carboxylic acid
1-({4-[5-(2-ethyl-4-biphenylyl)-1,2,4-oxadiazol-3-yl]phenyl}methyl)-1*H*-pyrazole-3-carboxylic acid
1-[(4-{5-[4-[(1-methylethyl)oxy]-3-(trifluoromethyl)phenyl]-1,2,4-oxa-diazol-3-yl}phenyl)methyl]-1H-pyrazole-3-carboxylic acid
1-({4-[5-(2-ethyl-2'-fluoro-4-biphenylyl)-1,2,4-oxadiazol-3-yl]phenyl}methyl)-1*H*-pyrazole-3-carboxylic acid
1-[(4-{5-[2-(trifluoromethyl)-4-biphenylyl]-1,2,4-oxadiazol-3-yl}phenyl)methyl]-1*H*-pyrazole-3-carboxylic acid
1-[(3-chloro-4-{5-[4-phenyl-5-(trifluoromethyl)-2-thienyl]-1,2,4-oxadiazol-3-yl}phenyl)methyl]-1H-pyrazole-3-carboxylic acid
1-[(2-chloro-4-{5-[4-phenyl-5-(trifluoromethyl)-2-thienyl]-1,2,4-oxadiazol-3-yl}phenyl)methyl]-1H-pyrazole-3-carboxylic acid
1-{[3-bromo-4-(5-{3-cyano-4-[(1-methylethyl)oxy]phenyl}-1,2,4-oxadiazol-3-yl)phenyl]methyl}-1H-pyrazole-3-carboxylic acid

and pharmaceutically acceptable salts thereof.

**4.** A compound according to any one of claims 1 to 3 for use in the treatment of conditions or disorders mediated by S1P1 receptors, wherein the condition or disorder is multiple sclerosis, autoimmune diseases, chronic inflammatory disorders, asthma, inflammatory neuropathies, arthritis, transplantation, Crohn's disease, ulcerative colitis, lupus erythematosis, psoriasis, ischemia-reperfusion injury, solid tumours, and tumour metastasis, diseases associated with angiogenesis, vascular diseases, pain conditions, acute viral diseases, inflammatory bowel conditions, or insulin and non-insulin dependent diabetes.

**5.** A compound for use in the treatment of conditions or disorders mediated by S1P1 receptors according to claim 4 wherein the condition is multiple sclerosis.

**6.** Use of a compound according to any one of claims 1 to 3 to manufacture a medicament for use in the treatment of conditions or disorders mediated by S1P1 receptors, wherein the condition or disorder is multiple sclerosis, autoimmune diseases, chronic inflammatory disorders, asthma, inflammatory neuropathies, arthritis, transplantation Crohn's disease, ulcerative colitis, lupus erythematosis, psoriasis, ischemia-reperfusion injury, solid tumours, and tumour metastasis, diseases associated with angiogenesis, vascular diseases, pain conditions, acute viral diseases, inflammatory bowel conditions, or insulin and non-insulin dependent diabetes.

**7.** Use according to claim 6 wherein the condition is multiple sclerosis.

**8.** A pharmaceutical composition comprising a compound according to any one of claims 1 to 3.

**Patentansprüche**

**1.** Eine Verbindung der Formel (I) oder ein pharmazeutisch verträgliches Salz davon

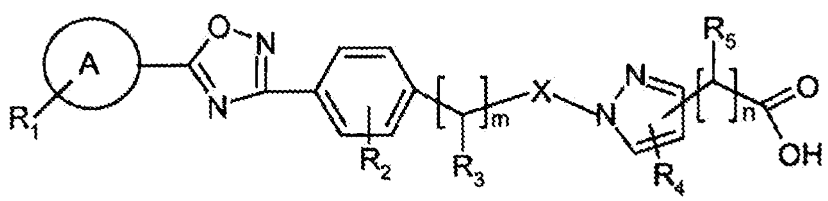

(I)

wobei

A ein Phenyl oder ein 5- oder 6-gliedriger Heteroarylring ist;
$R_1$ Wasserstoff ist oder bis zu drei Substitutenten, ausgewählt aus Halogen, $C_{(1-4)}$-Alkyl, $C_{(5-6)}$-Cycloalkyl, $C_{(3-6)}$-Cycloalkenyl, $C_{(1-4)}$-Alkoxy, Trifluomethoxy, Difluormethoxy, Trifluormethyl, Cyano, gegebenenfalls substituiertem Phenyl oder gegebenenfalls substituierten 5- oder 6-gliedrigen Heteroarylringen, darstellt;
$R_2$ Wasserstoff ist oder bis zu drei Substitutenten, ausgewählt aus Halogen, $C_{(1-4)}$-Alkyl, $C_{(1-4)}$-Alkoxy, Trifluormethoxy, Difluormethoxy, Trifluormethyl oder Cyano, darstellt;
$R_3$, $R_4$ und $R_5$ jeweils unabhängig ausgewählt sind aus Wasserstoff, Fluor und Methyl;
X ein gegebenenfalls substituierter $CH_2$-Rest oder eine direkte Bindung ist;
m 0 bis 1 ist; und
n 0 bis 2 ist;
wobei, wenn $R_1$ ein substituiertes Phenyl oder einen 5- oder 6-gliedrigen Heteroarylring umfasst, es mit bis zu drei Substitutenten, unabhängig ausgewählt aus Halogen, $C_{(1-4)}$-Alkyl, $C_{(1-4)}$-Alkoxy, Trifluormethoxy, Difluormethoxy, Trifluormethyl und Cyano, substituiert ist.

**2.** Eine Verbindung der Formel (I) oder ein pharmazeutisch verträgliches Salz davon, wobei

A gegebenenfalls substituiertes Pyridyl, Phenyl oder Thiophen ist; und/oder
$R_1$ gegebenenfalls substituiertes Phenol, Trifluorethyl, Trifluormethoxy, Chlor, Isopropoxy, Isobutyl, Methoxy, Cyano, Cyclohexyl, Cyclohexenyl oder Ethyl ist; und/oder
$R_2$ Wasserstoff, Chlor oder Brom ist; und/oder
$R_3$, $R_4$ und $R_5$ jeweils Wasserstoff sind; und/oder
X $CH_2$ ist; und/oder
m 0 ist; und/oder
n 0 ist.

**3.** Eine Verbindung, ausgewählt aus:

1-[(4-{5-[4-Phenyl-5-(trifluormethyl)-2-thienyl]-1,2,4-oxadiazol-3-yl}phenyl)methyl]-1*H*-pyrazol-4-carbonsäure,
1-[(4-{5-[4-Phenyl-5-(trifluormethyl)-2-thienyl]-1,2,4-oxadiazol-3-yl}phenyl)methyl]-1*H*-pyrazol-5-carbonsäure,
1-[(4-{5-[4-Phenyl-5-(trifluormethyl)-2-thienyl]-1,2,4-oxadiazol-3-yl}phenyl)methyl]-1*H*-pyrazol-3-carbonsäure,
1-[(4-{5-[4-(4-Fluorphenyl)-5-(trifluormethyl)-2-thienyl]-1,2,4-oxadiazol-3-yl}phenyl)methyl]-1*H*-pyrazol-3-carbonsäure,
1-{[4-(5-{5-Chlor-6-[(1-methylethyl)oxy]-3-pyridinyl}-1,2,4-oxadiazol-3-yl)phenyl]methyl}-1*H*-pyrazol-3-carbonsäure,
1-[(4-{5-[4-(Methyloxy)-3-(trifluormethyl)phenyl]-1,2,4-oxadiazol-3-yl}phenyl)methyl]-1*H*-pyrazol-3-carbonsäure,
1-{[4-(5-{3-Chlor-4-[(1-methylethyl)oxy]phenyl}-1,2,4-oxadiazol-3-yl)phenyl]methyl}-1*H*-pyrazol-3-carbonsäure,
1-{[4-(5-{3-Chlor-4-[(trifluormethyl)oxy]phenyl}-1,2,4-oxadiazol-3-yl)phenyl]methyl}-1*H*-pyrazol-3-carbonsäure,
1-{[4-(5-{3-Cyano-4-[(1-methylethyl)oxy]phenyl}-1,2,4-oxadiazol-3-yl)phenyl]methyl}-1*H*-pyrazol-3-carbonsäure,
1-[(4-{5-[2'-Fluor-2-(trifluormethyl)-4-biphenylyl]-1,2,4-oxadiazol-3-yl}phenyl)methyl]-1*H*-pyrazol-3-carbonsäure,
1-[(4-{5-[2'-Fluor-2-(trifluormethyl)-4-biphenylyl]-1,2,4-oxadiazol-3-yl}phenyl)methyl]-1*H*-pyrazol-3-carbonsäure,
1-[(4-{5-[4-Cyclohexyl-3-(trifluormethyl)phenyl]-1,2,4-oxadiazol-3-yl}phenyl)methyl]-1*H*-pyrazol-3-carbonsäure,
1-[(4-{5-[4-(1-Cyclohcxen-1-yl)-3-(trifluomiethyl)phenyl]-1,2,4-oxadiazol-3-yl}phenyl)methyl]-1*H*-pyrazol-3-carboosäure,
1-[(4-{5-[4-(2-Methylpropyl)-3-(trifluomethyl)phenyl]-1,2,4-oxadiazol-3-yl}phenyl)methyl]-1*H*-pyrazol-3-carbonsäure,
1-({4-[5-(2-Ethyl-4-biphenylyl)-1,2,4-oxadiazol-3-yl]phenyl}methyl)-1*H*-pyrazol-3-carbonsäure,
1-[(4-{5-[4-[(1-Methylethyl)oxy]-3-(trifluormethyl)phenyl]-1,2,4-oxadiazol-3-yl}phenyl)methyl]-1*H*-pyrazo1-3-carbonsäure,
1-({4-[5-(2-Ethyl-2'-fluor-4-biphenylyl)-1,2,4-oxadiazol-3-yl]phenyl}methyl)-1*H*-pyrazol-3-carbonsäure,
1-[(4-{5-[2-(Trifluormethyl)-4-biphenylyl]-1,2,4-oxadiazol-3-yl}phenyl)methyl]-1*H*-pyrazol-3-carbonsäure,
1-[(3-Chlor-4-{5-[4-phenyl-5-(trifluormethyl)-2-thienyl]-1,2,4-oxadiazal-3-yl}phenyl)methyl]-1*H*-pyrazol-3-carbonsäure,
1-[(2-Chlor-4-{5-[4-phenyl-5-(trifluormethyl)-2-thienyl]-1,2,4-oxadiazol-3-yl}phenyl)methyl]-1*H*-pyrazol-3-carbonsäure,
1-{[3-Brom-4-(5-{3-cyano-4-[(1-methylethyl)oxy]phenyl}-1,2,4-oxadiazol-3-yl)phenyl]methyl}-1*H*-pyrazol-3-carbonsäure

und pharmazeutisch verträgliche Salze davon.

**4.** Eine Verbindung gemäß einem der Ansprüche 1 bis 3 zur Verwendung bei der Behandlung von durch S1P1-Rezeptoren vermittelten Zuständen oder Störungen, wobei es sich bei dem Zustand oder der Störung um Multiple Sklerose, Autoimmunerkrankungen, chronische entzündliche Erkrankungen, Asthma, entzündliche Nervenleiden, Arthritis, Transplantation, Morbus Crohn, Colitis ulcerosa, Lupus erythematodes, Psoriasis, Ischämie-Reperfusionsschaden, feste Tumoren und Tumor-Metastasen, Erkrankungen, die mit Angiogenese in Zusammenhang stehen, Gefäßerkrankungen, Schmerzzustände, akute Viruserkrankungen, entzündliche Darmerkrankungen oder insulinabhängigen und nicht-insulinabhängigen Diabetes mellitus handelt.

5. Eine Verbindung zur Verwendung bei der Behandlung von durch S1P1-Rezeptoren vermittelten Zuständen oder Störungen gemäß Anspruch 4, wobei der Zustand Multiple Sklerose ist.

6. Verwendung einer Verbindung gemäß einem der Ansprüche 1 bis 3, um ein Medikament zur Verwendung bei der Behandlung von durch S1P1-Rezeptoren vermittelten Zuständen oder Störungen herzustellen, wobei es sich bei dem Zustand oder der Störung um Multiple Sklerose, Autoimmunerkrankungen, chronische entzündliche Erkrankungen, Asthma, entzündliche Nervenleiden, Arthritis, Transplantation, Morbus Crohn, Colitis ulcerosa, Lupus erythematodes, Psoriasis, Ischämie-Reperfusionsschaden, feste Tumoren und Tumor-Metastasen, Erkrankungen, die mit Angiogenese in Zusammenhang stehen, Gefüßerkrankungen, Schmerzzustände, akute Viruserkrankungen, entzündliche Darmerkrankungen oder insulinabhängigen und nicht-insulinabhängigen Diabetes mellitus handelt.

7. Verwendung gemäß Anspruch 6, wobei der Zustand Multiple Sklerose ist.

8. Ein Arzneimittel, umfassend eine Verbindung gemäß einem der Ansprüche 1 bis 3.

## Revendications

1. Composé de formule (I) ou un de ses sels pharmaceutiquement acceptables :

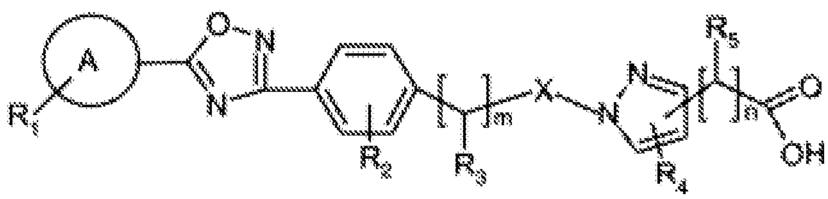

(I)

dans laquelle :

A représente un groupe phényle ou un noyau hétéroaryle penta- ou hexagonal ;
$R_1$ représente un atome d'hydrogène ou jusqu'à trois substituants choisis entre des substituants halogéno, alkyle en $C_1$ à $C_4$, cycloalkyle en $C_5$ ou $C_6$, cycloalcényle en $C_3$ à $C_6$, alkoxy en $C_1$ à $C_4$, trifluorométhoxy, difluorométhoxy, trifluorométhyle, cyano, phényle facultativement substitué ou des noyaux hétéroaryle penta- ou hexagonaux facultativement substitués ;
$R_2$ représente un atome d'hydrogène ou jusqu'à trois substituants choisis entre des substituants halogéno, alkyle en $C_1$ à $C_4$, alkoxy en $C_1$ à $C_4$, trifluorométhoxy, difluorométhoxy, trifluorométhyle et cyano ;
$R_3$, $R_4$ et $R_5$ sont choisis chacun indépendamment entre un atome d'hydrogène, des groupes fluoro et méthyle ;
X représente un groupe $CH_2$ facultativement substitué ou une liaison directe ;
m a une valeur de 0 à 1 ; et
n a une valeur de 0 à 2
où, lorsque $R_1$ comprend un groupe phényle substitué ou un noyau hétéroaryle penta- ou hexagonal substitué, il est substitué avec jusqu'à trois substituants choisis indépendamment entre des substituants halogéno, alkyle en $C_1$ à $C_4$, alkoxy en $C_1$ à $C_4$, trifluorométhoxy, difluorométhoxy, trifluorométhyle et cyano.

2. Composé de formule (I) ou un de ses sels pharmaceutiquement acceptables, dans lequel :

A représente un groupe pyridyle, phényle ou thiophène facultativement substitué ; et/ou
$R_1$ représente un groupe phényle facultativement substitué, trifluorométhyle, trifluorométhoxy, chloro, isopropoxy, isobutyle, méthoxy, cyano, cyclohexyle, cyclohexényle ou éthyle ; et/ou
$R_2$ représente un atome d'hydrogène, un groupe chloro ou bromo ; et/ou
$R_3$, $R_4$ et $R_5$ représentent chacun un atome d'hydrogène ; et/ou
X représente un groupe $CH_2$ ; et/ou

m est égal à 0 ; et/ou
n est égal à 0.

**3.** Composé choisi entre :

l'acide 1-[(4-{5-[4-phényl-5-(trifluorométhyl)-2-thiényl]-1,2,4-oxadiazole-3-yl}phényl)méthyl]-1*H*-pyrazole-4-carboxylique,
l'acide 1-[(4-{5-[4-phényl-5-(trifluorométhyl)-2-thiényl]-1,2,4-oxadiazole-3-yl}phényl)méthyl]-1*H*-pyrazole-5-carboxylique,
l'acide 1-[(4-{5-[4-phényl-5-(trifluorométhyl)-2-thiényl]-1,2,4-oxadiazole-3-yl}phényl)méthyl]-1*H*-pyrazole-3-carboxylique,
l'acide 1-[(4-{5-[4-(4-fluorophényl)-5-(trifluorométhyl)-2-thiényl]-1,2,4-oxadiazole-3-yl}phényl)méthyl]-1*H*-pyrazole-3-carboxylique,
l'acide 1-{[4-(5-{5-chloro-6-[(1-méthyléthyl)oxy]-3-pyridinyl}-1,2,4-oxadiazole-3-yl)phényl]méthyl]-1*H*-pyrazole-3-carboxylique,
l'acide 1-[(4-{5-[4-(méthyloxy)-3-(trifluorométhyl)phényl]-1,2,4-oxadiazole-3-yl}phényl)méthyl]-1*H*-pyrazole-3-carboxylique,
l'acide 1-{[4-(5-{3-chloro-4-[(1-méthyléthyl)oxy]phényl}-1,2,4-oxadiazole-3-yl)phényl]méthyl}-1*H*-pyrazole-3-carboxylique,
l'acide 1-{[4-(5-{3-chloro-4-[(trifluorométhyl)oxy]phényl}-1,2,4-oxadiazole-3-yl)phényl]méthyl}-1*H*-pyrazole-3-carboxylique,
l'acide 1-{[4-(5-{3-cyano-4-[(1-méthyléthyl)oxy]phényl}-1,2,4-oxadiazole-3-yl)phényl]méthyl}-1*H*-pyrazole-3-carboxylique,
l'acide 1-[(4-{5-[2'-fluoro-2-(trifluorométhyl)-4-biphénylyl]-1,2,4-oxadiazole-3-yl}phényl)méthyl]-1H-pyrazole-3-carboxylique,
l'acide 1-[(4-{5-[2'-fluoro-2-(trifluorométhyl)-4-biphénylyl]-1,2,4-oxadiazole-3-yl}phényl)méthyl]-1H-pyrazole-3-carboxylique,
l'acide 1-[(4-{5-[4-cyclohexyl-3-(trifluorométhyl)phényl]-1,2,4-oxadiazole-3-yl}phényl)méthyl]-1H-pyrazole-3-carboxylique,
l'acide 1-[(4-{5-[4-(1-cyclohexène-1-yl)-3-(trifluorométhyl)phényl]-1,2,4-oxadiazole-3-yl}phényl)méthyl]-1H-pyrazole-3-carboxylique,
l'acide 1-[(4-{5-[4-(2-méthylpropyl)-3-(trifluorométhyl)-phényl]-1,2,4-oxadiazole-3-yl}phényl)méthyl]-1H-pyrazole-3-carboxylique,
l'acide 1-({4-[5-(2-éthyl-4-biphénylyl)-1,2,4-oxadiazole-3-yl]phényl}méthyl)-1*H*-pyrazole-3-carboxylique,
l'acide 1-[(4-{5-[4-[(1-méthyléthyl)oxy]-3-(trifluorométhyl)-phényl]-1,2,4-oxadiazole-3-yl}phényl)méthyl]-1*H*-pyrazole-3-carboxylique,
l'acide 1-({4-[5-(2-éthyl-2'-fluoro-4-biphénylyl)-1,2,4-oxadiazole-3-yl]phényl}méthyl)-1*H*-pyrazole-3-carboxylique,
l'acide 1-[(4-{5-[2-(trifluorométhyl)-4-biphénylyl]-1,2,4-oxadiazole-3-yl}phényl)méthyl]-1*H*-pyrazole-3-carboxylique,
l'acide 1-[(3-chloro-4-{5-[4-phényl-5-(trifluorométhyl)-2-thiényl]-1,2,4-oxadiazole-3-yl}phényl)méthyl]-1H-pyrazole-3-carboxylique,
l'acide 1-[(2-chloro-4-{5-[4-phényl-5-(trifluorométhyl)-2-thiényl]-1,2,4-oxadiazole-3-yl}phényl)méthyl]-1H-pyrazole-3-carboxylique,
l'acide 1-{[3-bromo-4-(5-{3-cyano-4-[(1-méthyléthyl)oxy]-phényl}-1,2,4-oxadiazole-3-yl)phényl]méthyl}-1H-pyrazole-3-carboxylique,
et leurs sels pharmaceutiquement acceptables.

**4.** Composé suivant l'une quelconque des revendications 1 à 3, destiné à être utilisé dans le traitement d'affections ou de troubles à médiation par les récepteurs S1P1,
l'affection ou le trouble étant la sclérose en plaques, une maladie auto-immune, un trouble inflammatoire chronique, l'asthme, une neuropathie inflammatoire, l'arthrite, une transplantation, la maladie de Crohn, la colite ulcérative, le lupus érythémateux, le psoriasis, une lésion de reperfusion ischémique, une tumeur solide ou une métastase tumorale, une maladie associée à l'angiogenèse, une maladie vasculaire, une état douloureux, une maladie virale aiguë, une affection intestinale inflammatoire ou le diabète insulinodépendant ou non insulinodépendant.

**5.** Composé destiné à être utilisé dans le traitement d'affections ou de troubles à médiation par les récepteurs S1P1 suivant la revendication 4, l'affection étant la sclérose en plaques.

**6.** Utilisation d'un composé suivant l'une quelconque des revendications 1 à 3, pour la production d'un médicament destiné à être utilisé dans le traitement d'affections ou de troubles à médiation par les récepteurs S1P1, dans laquelle l'affection ou le trouble est la sclérose en plaques, une maladie auto-immune, un trouble inflammatoire chronique, l'asthme, une neuropathie inflammatoire, l'arthrite, une transplantation, la maladie de Crohn, la colite ulcérative, le lupus érythémateux, le psoriasis, une lésion de reperfusion ischémique, une tumeur solide ou une métastase tumorale, une maladie associée à l'angiogenèse, une maladie vasculaire, une état douloureux, une maladie virale aiguë, une affection intestinale inflammatoire ou le diabète insulinodépendant ou non insulinodépendant.

**7.** Utilisation suivant la revendication 6, dans laquelle l'affection est la sclérose en plaques.

**8.** Composition pharmaceutique comprenant un composé suivant l'une quelconque des revendications 1 à 3.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 11080026 A
- WO 03105771 A
- WO 05058848 A
- WO 06047195 A
- WO 06100633 A
- WO 06115188 A
- WO 06131336 A
- WO 2007024922 A
- WO 07116866 A
- WO 05032465 A
- WO 04103279 A
- WO 2003061567 A2
- WO 2003061567 A
- WO 199702244 A
- WO 2007039177 A
- WO 200558848 A


### Non-patent literature cited in the description


- **Okamoto et al.** *J Biol Chem,* 1998, vol. 273 (42), 27104
- **Sanchez ; Hla.** *J Cell Biochem,* 2004, vol. 92, 913
- **Pyne ; Pyne.** *Biochem J.,* 2000, vol. 349, 385
- **Chun et al.** *Pharmacological Reviews,* 2002, vol. 54, 265
- **Sanchez ; Hla.** *J Cellular Biochemistry,* 2004, vol. 92, 913
- **Kluk ; Hla.** *Biochem et Biophysica Acta,* 2002, vol. 1582, 72
- **Sanchez ; Hla.** *J Cellular Biochem,* 2004, vol. 92, 913
- **Rosen ; Goetzl.** *Nat Rev Immunol.,* 2005, vol. 5, 560
- **Brinkman et al.** *JBC,* 2003, vol. 277, 21453
- **Fujino et al.** *J Pharmacol Exp Ther,* 2003, vol. 305, 70
- **Webb et al.** *J Neuroimmunol,* 2004, vol. 153, 108
- **Rausch et al.** *J Magn Reson Imaging,* 2004, vol. 20, 16
- **Chiba et al.** *J Immunology,* 1998, vol. 160, 5037
- **Forrest et al.** *J Pharmacol Exp Ther,* 2004, vol. 309, 758
- **Sanna et al.** *JBC,* 2004, vol. 279, 13839
- **Graler ; Goetzl.** *FASEB J,* 2004, vol. 18, 551
- **Matloubian et al.** *Nature,* 2004, vol. 427, 355
- **Jo et al.** *Chem Biol,* 2005, vol. 12, 703
- **Allende et al.** *Blood,* 2003, vol. 102, 3665
- **Singelton et al.** *FASEB J,* 2005, vol. 19, 1646
- **Wei wt.** *Nat. Immunology,* 2005, vol. 6, 1228
- **Brinkman et al.** *JBC,* 2002, vol. 277, 21453
- **Mandala et al.** *Science,* 2002, vol. 296, 346
- **Fujino et al.** *J Pharmacology and Experimental Therapeutics,* 2003, vol. 305, 45658
- **Brinkman et al.** *American J Transplantation,* 2004, vol. 4, 1019
- **Webb et al.** *J Neuroimmunology,* 2004, vol. 153, 108
- **Morris et al.** *EurJ Immunol,* 2005, vol. 35, 3570
- **Chiba.** *Pharmacology and Therapeutics,* 2005, vol. 108, 308
- **Kahan et al.** *Transplantation,* 2003, vol. 76, 1079
- **Kappos et al.** *New Eng J Medicine,* 2006, vol. 335, 1124
- **Hale et al.** *Bioorganic & Medicinal Chemistry Letters,* 2004, vol. 14, 3501
- **Koyrakh et al.** *American J Transplantation,* 2005, vol. 5, 529
- **Guthrie ; Fink.** *Methods in Enzymology,* 1991, vol. 194